# EUROPEAN PATENT SPECIFICATION

(11) **EP 1 581 467 B1**
(45) Date of publication and mention of the grant of the patent: **12.10.2016**
(21) Application number: 03812849.2
(22) Date of filing: 09.12.2003
(51) Int. Cl.: C07C 45/00, C07C 6/00, C07C 21/00, C07F 9/02, C07F 9/50

(54) **LIGANDS FOR METALS AND IMPROVED METAL-CATALYZED PROCESSES BASED THEREON**
LIGANDEN FÜR METALLE UND DARAUF BERUHENDE VERBESSERTE METALLKATALYSIERTE VERFAHREN
LIGANDS POUR MÉTAUX ET PROCESSUS A CATALYSE PAR MÉTAUX AMÉLIORÉS BASÉS SUR CES LIGANDS

(30) Priority: 09.12.2002 US 431870 P; 03.03.2003 US 451562 P
(43) Date of publication of application: 05.10.2005
(60) Divisional application: 12177241.2
(73) Proprietor: MASSACHUSETTS INSTITUTE OF TECHNOLOGY, Cambridge, MA 02139 (US)
(72) Inventor: BUCHWALD, Stephen, L., Newton, MA 02558 (US); HUANG, Xiaohua, Malden, MA 02148 (US); ZIM, Danilo, Campinas, 13090-600 (BR)
(74) Representative: Atkinson, Jonathan David Mark
(86) International application number: PCT/US2003/038945
(87) International publication number: WO 2004/052939

(56) References cited:
- WO-A-03/006420
- US-A- 5 663 426
- US-B1- 6 395 916
- LEE S ET AL: "Palladium-catalyzed synthesis of arylamines from aryl halides and lithium bis(trimethylsilyl)amide as an ammonia equivalent" ORGANIC LETTERS, ACS, WASHINGTON, DC, vol. 3, no. 17, 1 January 2001 (2001-01-01), pages 2729-2732, XP002964608 ISSN: 1523-7060
- STAUFFER SHAUN R ET AL: "Palladium-Catalyzed Arylation of Ethyl Cyanoacetate. Fluorescence Resonance Energy Transfer as a Tool for Reaction Discovery" JOURNAL OF THE AMERICAN CHEMICAL SOCIETY, US, vol. 123, no. 19, 1 January 2001 (2001-01-01), pages 4641-4642, XP002186737 ISSN: 0002-7863
- J.STAMBULI,SH. STAUFFER: "screening of homgeneous catalysts by fluorescence resonance energy transfer." JOURNAL OF THE AMERICAN CHEMICAL SOCIETY., vol. 123, no. 11, 2001, pages 2677-2678, XP002486659 USAMERICAN CHEMICAL SOCIETY, WASHINGTON, DC.
- E.STRIETER ET AL.: "insights into the origin of high activity and stability of catalysts derived from bulky,electron-rich monophosphinobiaryl ligands in the pd-catalysed c-n bond formation." JOURNAL OF THE AMERICAN CHEMICAL SOCIETY., vol. 125, no. 46, 2003, pages 13978-13980, XP002486660 USAMERICAN CHEMICAL SOCIETY, WASHINGTON, DC.
- DATABASE CAPLUS [Online] 1994 FABBRI D. ET AL: 'Binaphthyl-substituted chiral phosphines and oxides from binaphtophospholes and nucleophiles', XP002903242 Retrieved from STN Database accession no. 1994:508962 & SYNTHETIC COMMUNICATIONS vol. 24, no. 9, 1994, pages 1271 - 1278
- DATABASE CAPLUS [Online] 2000 OLD ET AL: 'Efficient Palladium-Catalyzed N-Arylation of Indoles', XP002903243 Retrieved from STN Database accession no. 2000:248593 & ORGANIC LETTERS vol. 2, no. 10, 2000, pages 1403 - 1406
- DATABASE CAPLUS [Online] 2000 TOMORI ET AL: 'An Improved Synthesis of Functionalized Biphenyl-Based Phosphine Ligands', XP002903244 Retrieved from STN Database accession no. 2000:523451 & JOURNAL OF ORGANIC CHEMISTRY vol. 65, no. 17, 2000, pages 5334 - 5341
- DATABASE CAPLUS [Online] 1991 VAN DEN WINKEL ET AL: 'Investigations of a highly crowded phosphino-substituted biphenyl: a precursor for a lambda3,lambda5-diphosphaphenanthrene', XP002903245 Retrieved from STN Database accession no. 1991:229033 & HETEROATOM CHEMISTRY vol. 2, no. 1, 1991, pages 17 - 28
- DATABASE CAPLUS [Online] 2003 HUANG ET AL: 'Expanding Pd-Catalyzed C-N Bond-Forming Processes: The First Amidation of Aryl Sulfonates, Aqueous Amination, and Complementarity with Cu-Catalyzed Reactions', XP002903246 Retrieved from STN Database accession no. 2003:344190 & JOURNAL OF THE AMERICAN CHEMICAL SOCIETY vol. 125, no. 22, 2003, pages 6653 - 6655
- HUANG X BUCHWALD S L: "New ammonia equivalents for the Pd-catalyzed amination of aryl halides", ORGANIC LETTERS, AMERICAN CHEMICAL SOCIETY, US LNKD- DOI:10.1021/OL0166808, vol. 3, no. 21, 1 September 2001 (2001-09-01), pages 3417-3419, XP002958148, ISSN: 1523-7060
- KAZUHIKO SEMBA ET AL: "Arylboration of Alkenes by Cooperative Palladium/Copper Catalysis", JOURNAL OF THE AMERICAN CHEMICAL SOCIETY, vol. 136, no. 21, 28 May 2014 (2014-05-28) , pages 7567-7570, XP055268364, US ISSN: 0002-7863, DOI: 10.1021/ja5029556

## Description

Transition metal catalyst complexes play important roles in many areas of chemistry, including the preparation of polymers and pharmaceuticals. The properties of these catalyst complexes are recognized to be influenced by both the characteristics of the metal and those of the ligands associated with the metal atom. For example, structural features of the ligands can influence reaction rate, regioselectivity, and stereoselectivity. Bulky ligands can be expected to slow reaction rate; electron-withdrawing ligands, in coupling reactions, can be expected to slow oxidative addition to, and speed reductive elimination from, the metal center; and electron-rich ligands, in coupling reactions, conversely, can be expected to speed oxidative addition to, and slow reductive elimination from, the metal center.

In many cases, the oxidative addition step in the accepted mechanism of a coupling reaction is deemed to be rate limiting. Therefore, adjustments to the catalytic system as a whole that increase the rate of the oxidative addition step should increase overall reaction rate. Additionally, the rate of oxidative addition of a transtion metal catalyst to the carbon-halogen bond of an aryl halide is known to decrease as the halide is varied from iodide to bromide to chloride, all other factors being equal. Because of this fact, the more stable, lower molecular weight, and arguably more easy to obtain, members of the group consisting of reactive organic halides - the chlorides - are the poorest substrates for traditional transition metal catalyzed coupling reactions and the like.

To date, the best halogen-containing substrates for transtion metal catalyzed carbon-heteroatom and carbon-carbon bond forming reactions have been the iodides. Bromides have often been acceptable substrates, but typically required higher temperatures, longer reaction times, and gave lower yields of products.

Tomori et al, J. Org. Chem. 2000, 65, 5334-5341 describe functionalised dicyclohexyl- and di-*tert*-butylphosphinobiphenyl ligands.

### Summary of the Invention

One aspect of the present invention relates to novel ligands for transition metals. This disclosure also relates to the use of catalysts comprising these ligands in various transition-metal-catalyzed carbon-heteroatom and carbon-carbon bond-forming reactions. The subject methods provide improvements in many features of transition-metal-catalyzed reactions, including the range of suitable substrates, number of catalyst turnovers, reaction conditions, and efficiency.

More specifically, unexpected, pioneering improvements have been realized in transition metal-catalyzed: aryl amination reactions; aryl amidation reactions; Suzuki couplings to give biaryl products; and α-arylations of ketones. For example, the use of an alcohol, e.g., *tert*-butanol, as the solvent in certain methods of the present disclosure allows the use of substrates comprising functional groups that were heretofore precluded because the functional groups were not sufficiently stable to survive the reaction conditions or because they would otherwise interfere with the transition-metal-catalyzed methods. Further, the ligands and methods of the present disclosure enable the efficient use of aryl sulfonates, e.g., aryl tosylates, in the aforementioned reactions.

Additionally, the ligands and methods of the present disclosure enable for the first time transformations utilizing aryl bromides or chlorides to proceed efficiently at room temperature. Furthermore, the ligands and methods of the present disclosure enable the aforementioned reactions to occur at synthetically useful rates using extremely small amounts of catalyst, e.g., 0.000001 mol% relative to the limiting reagent.

### Brief Description of the Figures

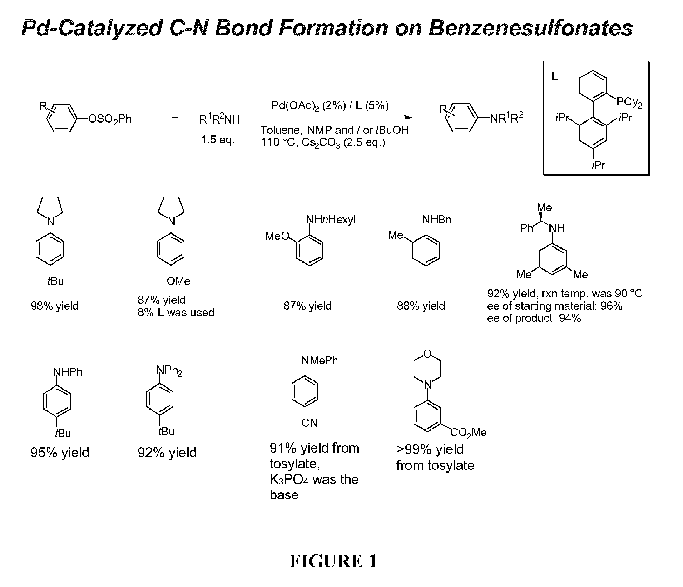

**Figure 1** depicts various embodiments of a carbon-nitrogen bond-forming method of the present invention, using various ligands and various benzenesulfonates.

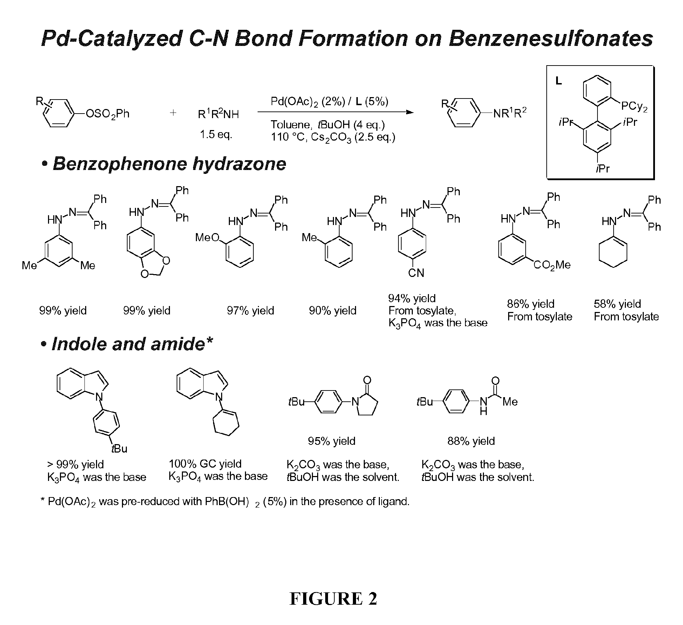

**Figure 2** depicts various embodiments of a carbon-nitrogen bond-forming method of the present invention, using various ligands and various benzenesulfonates.

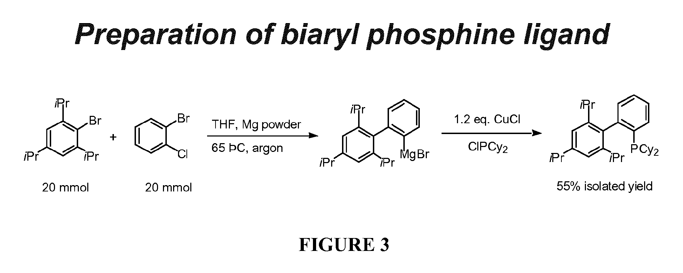

**Figure 3** depicts a synthetic protocol for the preparation of a ligand of the present invention.

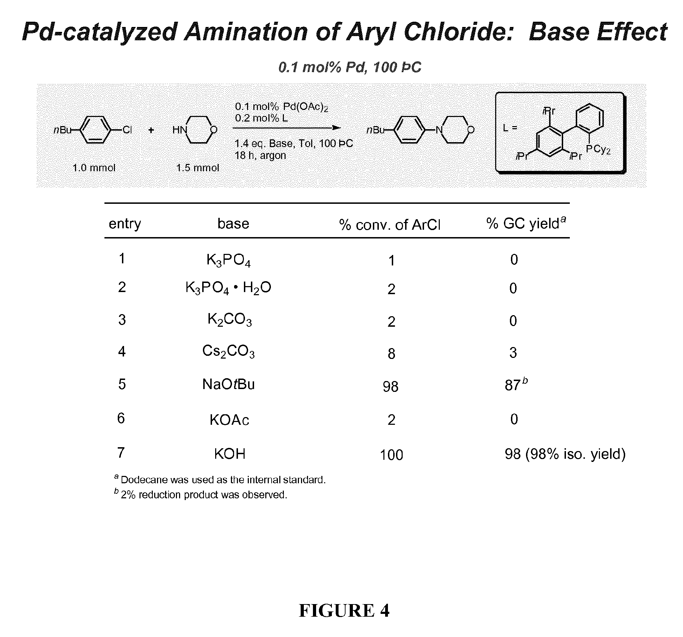

**Figure 4** depicts various embodiments of a carbon-nitrogen bond-forming method of the present invention, using a preferred ligand of the present invention and 4-butylphenyl chloride, as a function of the base used.

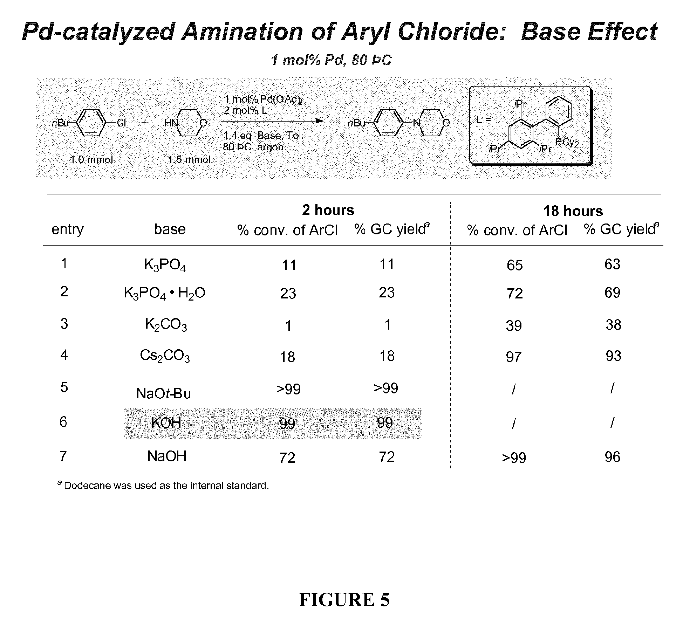

**Figure 5** depicts various embodiments of a carbon-nitrogen bond-forming method of the present invention, using a preferred ligand of the present invention and 4-butylphenyl chloride, as a function of the base used.

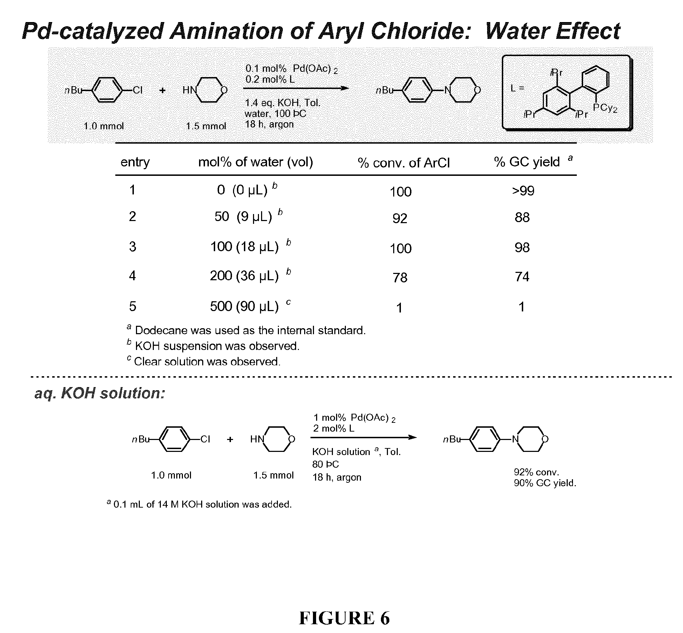

**Figure 6** depicts various embodiments of a carbon-nitrogen bond-forming method of the present invention, using a preferred ligand of the present invention and 4-butylphenyl chloride, as a function of the amount of water in the reaction mixture.

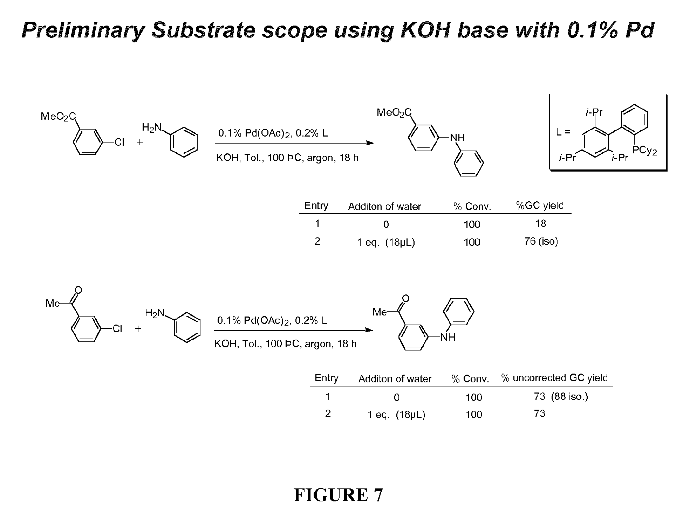

**Figure 7** depicts various embodiments of a carbon-nitrogen bond-forming method of the present invention, using a preferred ligand of the present invention and various aryl chlorides, as a function of the amount of water in the reaction mixture.
**Figure 8** depicts various embodiments of a carbon-nitrogen bond-forming method of the present invention, using a preferred ligand of the present invention and 4-butylphenyl chloride, as a function of the reaction time.
**Figure 9** depicts various embodiments of a carbon-carbon bond-forming method of the present invention, using as ligand for palladium various (2',4',6'-tri-isopropylbiphenyl)di(alkyl)phosphines, 4-*tert*-butylphenyl tosylate, and phenyl boronic acid. *See* Example 66.

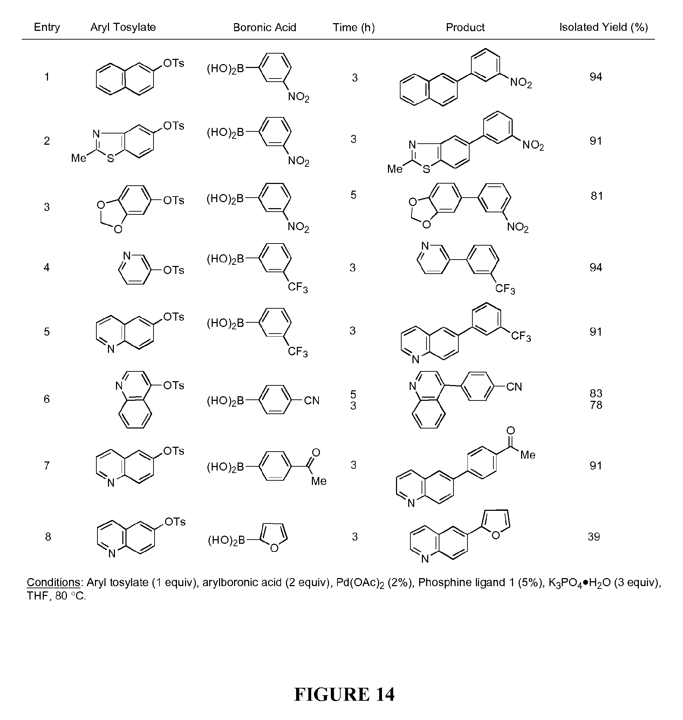

**Figure 10** depicts various embodiments of a carbon-carbon bond-forming method of the present invention, using as ligand for palladium (2',4',6'-tri-isopropylbiphenyl)dicyclohexylphosphine, various aryl tosylates, and various aryl boronic acids. *See* Example 66 and Figure 14.
**Figure 11** depicts various embodiments of a carbon-carbon bond-forming method of the present invention, using as ligand for palladium (2',4',6'-tri-isopropylbiphenyl)dicyclohexylphosphine, various aryl tosylates, and various aryl boronic acids. *See* Example 66 and Figure 14.
**Figure 12** depicts various embodiments of a carbon-carbon bond-forming method of the present invention, using as ligand for palladium (2',4',6'-tri-isopropylbiphenyl)dicyclohexylphosphine, various aryl tosylates, and various aryl boronic acids. *See* Example 66 and Figure 14.
**Figure 13** depicts various embodiments of a carbon-carbon bond-forming method of the present invention, using as ligand for palladium various (2',4',6'-tri-isopropylbiphenyl)di(alkyl)phosphines, various aryl tosylates, and various aryl boronic acids. *See* Example 66 and Figure 14.
**Figure 14** depicts various embodiments of a carbon-carbon bond-forming method of the present invention, using as ligand for palladium (2',4',6'-tri-isopropylbiphenyl)dicyclohexylphosphine, various aryl tosylates, and various aryl boronic acids. *See* Example 66 and Figure 14.

### Detailed Description of the Invention

### Ligands of the Present Invention

According to a first aspect, the present invention provides a ligand represented by structure II: 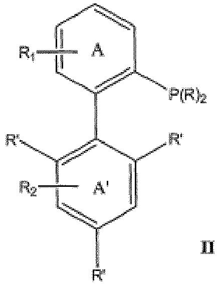 wherein: R is independently for each occurrence cycloalkyl; wherein R' represents isopropyl; the A and A' rings of the biphenyl core independently may be unsubstituted or substituted with R₁ and R₂, respectively, any number of times up to the limitations imposed by stability and the rules of valence; R₁ and R₂, when present, are selected independently for each occurrence from the group consisting of alkyl, cycloalkyl, halogen, -SiR₃, and -(CH₂)ₘ-R₈₀; R₈₀ represents independently for each occurrence cycloalkyl or aryl; m is independently for each occurrence an integer in the range 0 to 8 inclusive; and the ligand, when chiral, is a mixture of enantiomers or a single enantiomer.

In certain embodiments, the ligands of the present invention are represented by structure **II** and the attendant definitions, wherein R₁ is absent; and R₂ is absent.

In certain embodiments, the ligands of the present invention are represented by structure **II** and the attendant definitions, wherein R represents independently for each occurrence cyclohexyl or cyclopropyl.

In certain embodiments, the ligands of the present invention are represented by structure **II** and the attendant definitions, wherein R represents independently for each occurrence cyclohexyl.

In certain embodiments, the ligands of the present invention are represented by structure **II** and the attendant definitions, wherein R₁ is absent; R₂ is absent; and R represents independently for each occurrence cyclohexyl or cyclopropyl.

In certain embodiments, the ligands of the present invention are represented by structure **II** and the attendant definitions, wherein R₁ is absent; R₂ is absent; and R represents independently for each occurrence cyclohexyl.

In certain embodiments, the ligands of the present invention are represented by structure **II** and the attendant definitions, wherein R₁ is absent; R₂ is absent; R represents independently for each occurrence cyclohexyl; and R' represents independently for each occurrence isopropyl.

The ligands of the present invention can be used to produce synthetic intermediates that, after being subjected to additional methods known in the art, are transformed to desired end products, e.g., lead compounds in medicinal chemistry programs, pharmaceuticals, insecticides, antivirals and antifungals. Furthermore, the ligands of the present invention may be used to increase the efficiency of and/or shorten established routes to desired end products, e.g., lead compounds in medicinal chemistry programs, pharmaceuticals, insecticides, antivirals and antifungals.

### II. Definitions

For convenience, before further description of the present invention, certain terms employed in the specification, examples, and appended claims are collected here.

The terms "biphenyl" and "binaphthylene" refer to the ring systems below. The numbers around the peripheries of the ring systems are the positional numbering systems used herein. Likewise, the capital letters contained within the individual rings of the ring systems are the ring descriptors used herein.

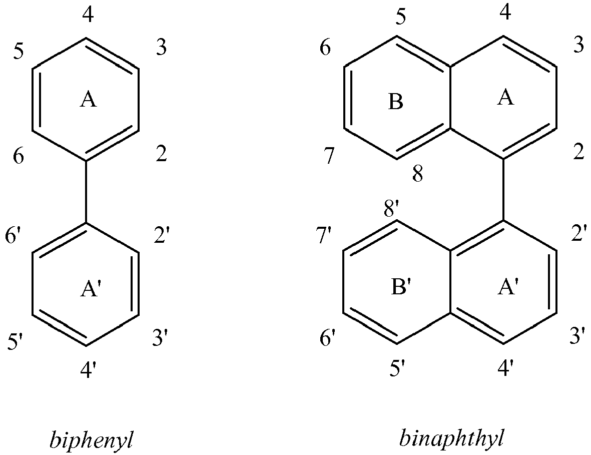

The term "substrate aryl group" refers to an aryl group containing an electrophilic atom which is susceptible to the subject cross-coupling reaction, e.g., the electrophilic atom bears a leaving group. In reaction scheme 1, the substrate aryl is represented by ArX, and X is the leaving group. The aryl group, Ar, is said to be substituted if, in addition to X, it is substituted at yet other positions. The substrate aryl group can be a single ring molecule, or can be a component of a larger molecule.

The term "nucleophile" is recognized in the art, and as used herein means a chemical moiety having a reactive pair of electrons.

The term "electrophile" is art-recognized and refers to chemical moieties which can accept a pair of electrons from a nucleophile as defined above. Electrophilic moieties useful in the method of the present invention include halides and sulfonates.

The terms "electrophilic atom", "electrophilic center" and "reactive center" as used herein refer to the atom of the substrate aryl moiety which is attacked by, and forms a new bond to the nucleophilic heteroatom of the hydrazine and the like. In most (but not all) cases, this will also be the aryl ring atom from which the leaving group departs.

The term "electron-withdrawing group" is recognized in the art, and denotes the tendency of a substituent to attract valence electrons from neighboring atoms, i.e., the substituent is electronegative with respect to neighboring atoms. A quantification of the level of electron-withdrawing capability is given by the Hammett sigma (s) constant. This well known constant is described in many references, for instance, J. March, Advanced Organic Chemistry, McGraw Hill Book Company, New York, (1977 edition) pp. 251-259. The Hammett constant values are generally negative for electron donating groups (s[P] = - 0.66 for NH₂) and positive for electron withdrawing groups (s[P] = 0.78 for a nitro group), s[P] indicating para substitution. Exemplary electron-withdrawing groups include nitro, ketone, aldehyde, sulfonyl, trifluoromethyl, -CN, chloride, and the like. Exemplary electron-donating groups include amino, methoxy, and the like.

The term "reaction product" means a compound which results from the reaction of the hydrazine or the like and the substrate aryl group. In general, the term "reaction product" will be used herein to refer to a stable, isolable aryl ether adduct, and not to unstable intermediates or transition states.

The term "catalytic amount" is recognized in the art and means a substoichiometric amount of reagent relative to a reactant. As used herein, a catalytic amount means from 0.0001 to 90 mole percent reagent relative to a reactant, more preferably from 0.001 to 50 mole percent, still more preferably from 0.01 to 10 mole percent, and even more preferably from 0.1 to 5 mole percent reagent to reactant.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 30 or fewer carbon atoms in its backbone (e.g., C₁-C₃₀ for straight chain, C₃-C₃₀ for branched chain), and more preferably 20 or fewer. Likewise, preferred cycloalkyls have from 3-10 carbon atoms in their ring structure, and more preferably have 5, 6 or 7 carbons in the ring structure.

Moreover, the term "alkyl" (or "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, a halogen, a hydroxyl, a carbonyl (such as a carboxyl, an ester, a formyl, or a ketone), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF₃, -CN, and the like.

The term "aralkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

The term "aryl" as used herein includes 5-, 6- and 7-membered single-ring aromatic groups that may include from zero to four heteroatoms, for example, benzene, naphthalene, anthracene, pyrene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics". The aromatic ring can be substituted at one or more ring positions with such substituents as described above, for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls.

The abbreviations Me, Et, Ph, Tf, Nf, Ts, Ms, and dba represent methyl, ethyl, phenyl, trifluoromethanesulfonyl, nonafluorobutanesulfonyl, *p*-toluenesulfonyl, methanesulfonyl, and dibenzylideneacetone, respectively. A more comprehensive list of the abbreviations utilized by organic chemists of ordinary skill in the art appears in the first issue of each volume of the *Journal of Organic Chemistry*; this list is typically presented in a table entitled Standard List of Abbreviations. The abbreviations contained in said list, and all abbreviations utilized by organic chemists of ordinary skill in the art are hereby incorporated by reference.

The terms *ortho, meta* and *para* apply to 1,2-, 1,3- and 1,4-disubstituted benzenes, respectively. For example, the names 1,2-dimethylbenzene and *ortho*-dimethylbenzene are synonymous.

The terms "heterocyclyl" or "heterocyclic group" refer to 3- to 10-membered ring structures, more preferably 3- to 7-membered rings, whose ring structures include one to four heteroatoms. Heterocycles can also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The terms "polycyclyl" or "polycyclic group" refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle can be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle", as used herein, refers to an aromatic or non-aromatic ring in which each atom of the ring is carbon.

The term "heteroatom" as used herein means an atom of any element other than carbon or hydrogen. Preferred heteroatoms are nitrogen, oxygen, sulfur and phosphorous.

As used herein, the term "nitro" means -NO₂; the term "halogen" designates -F,-Cl, -Br or -I; the term "sulfhydryl" means -SH; the term "hydroxyl" means -OH; and the term "sulfonyl" means -SO₂-.

The terms "amine" and "amino" are art recognized and refer to both unsubstituted and substituted amines, e.g., a moiety that can be represented by the general formula: 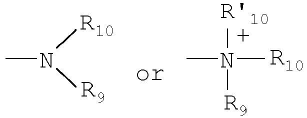 wherein R₉, R₁₀ and R'₁₀ each independently represent a hydrogen, an alkyl, an alkenyl, -(CH₂)ₘ-R₈, or R₉ and R₁₀ taken together with the N atom to which they are attached complete a heterocycle having from 4 to 8 atoms in the ring structure; R₈ represents an aryl, a cycloalkyl, a cycloalkenyl, a heterocycle or a polycycle; and m is zero or an integer in the range of 1 to 8. In preferred embodiments, only one of R₉ or R₁₀ can be a carbonyl, e.g., R₉, R₁₀ and the nitrogen together do not form an imide. In even more preferred embodiments, R9 and R10 (and optionally R'10) each independently represent a hydrogen, an alkyl, an alkenyl, or - (CH2)m-R8. Thus, the term "alkylamine" as used herein means an amine group, as defined above, having a substituted or unsubstituted alkyl attached thereto, i.e., at least one of R9 and R10 is an alkyl group.

The term "acylamino" is art-recognized and refers to a moiety that can be represented by the general formula: 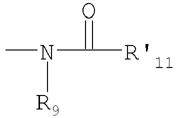 wherein R₉ is as defined above, and R'₁₁ represents a hydrogen, an alkyl, an alkenyl or -(CH₂)ₘ-R₈, where m and R₈ are as defined above.

The term "amido" is art recognized as an amino-substituted carbonyl and includes a moiety that can be represented by the general formula: 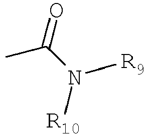 wherein R₉, R₁₀ are as defined above. Preferred embodiments of the amide will not include imides which may be unstable.

The term "alkylthio" refers to an alkyl group, as defined above, having a sulfur radical attached thereto. In preferred embodiments, the "alkylthio" moiety is represented by one of -S-alkyl, -S-alkenyl, -S-alkynyl, and -S-(CH₂)ₘ-R₈, wherein m and R₈ are defined above. Representative alkylthio groups include methylthio, ethyl thio, and the like.

The term "carbonyl" is art recognized and includes such moieties as can be represented by the general formula: 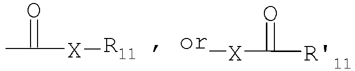 wherein X is a bond or represents an oxygen or a sulfur, and R₁₁ represents a hydrogen, an alkyl, an alkenyl, -(CH₂)ₘ-R₈ or a pharmaceutically acceptable salt, R'₁₁ represents a hydrogen, an alkyl, an alkenyl or -(CH₂)ₘ-R₈, where m and R₈ are as defined above. Where X is an oxygen and R₁₁ or R'₁₁ is not hydrogen, the formula represents an "ester". Where X is an oxygen, and R₁₁ is as defined above, the moiety is referred to herein as a carboxyl group, and particularly when R₁₁ is a hydrogen, the formula represents a "carboxylic acid". Where X is an oxygen, and R'₁₁ is hydrogen, the formula represents a "formate". In general, where the oxygen atom of the above formula is replaced by sulfur, the formula represents a "thiolcarbonyl" group. Where X is a sulfur and R₁₁ or R'₁₁ is not hydrogen, the formula represents a "thiolester." Where X is a sulfur and R₁₁ is hydrogen, the formula represents a "thiolcarboxylic acid." Where X is a sulfur and R₁₁' is hydrogen, the formula represents a "thiolformate." On the other hand, where X is a bond, and R₁₁ is not hydrogen, the above formula represents a "ketone" group. Where X is a bond, and R₁₁ is hydrogen, the above formula represents an "aldehyde" group.

The terms "alkoxyl" or "alkoxy" as used herein refers to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, - O-alkynyl, -O-(CH₂)ₘ-R₈, where m and R₈ are described above.

The term "sulfonate" is art recognized and includes a moiety that can be represented by the general formula: 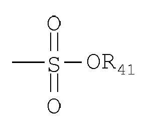 in which R₄₁ is an electron pair, hydrogen, alkyl, cycloalkyl, or aryl.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The term "sulfate" is art recognized and includes a moiety that can be represented by the general formula: 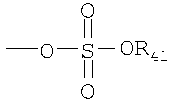 in which R₄₁ is as defined above.

The term "sulfonamido" is art recognized and includes a moiety that can be represented by the general formula: 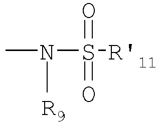 in which R₉ and R'₁₁ are as defined above.

The term "sulfamoyl" is art-recognized and includes a moiety that can be represented by the general formula: 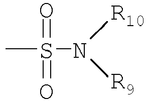 in which R₉ and R₁₀ are as defined above.

The terms "sulfoxido" or "sulfinyl", as used herein, refers to a moiety that can be represented by the general formula: 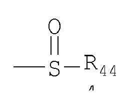 in which R₄₄ is selected from the group consisting of hydrogen, alkyl, alkenyl, alkynyl, cycloalkyl, heterocyclyl, aralkyl, or aryl.

A "phosphoryl" can in general be represented by the formula: 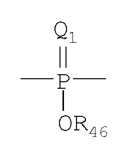 wherein Q₁ represented S or O, and R₄₆ represents hydrogen, a lower alkyl or an aryl. When used to substitute, e.g., an alkyl, the phosphoryl group of the phosphorylalkyl can be represented by the general formula: 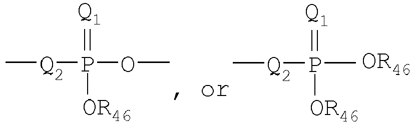 wherein Q₁ represented S or O, and each R₄₆ independently represents hydrogen, a lower alkyl or an aryl, Q₂ represents O, S or N. When Q₁ is an S, the phosphoryl moiety is a "phosphorothioate".

A "phosphoramidite" can be represented in the general formula: 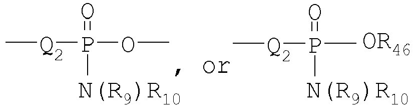 wherein R₉ and R₁₀ are as defined above, and Q₂ represents O, S or N.

A "phosphonamidite" can be represented in the general formula: 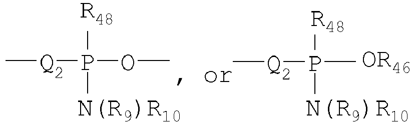 wherein R₉ and R₁₀ are as defined above, Q₂ represents O, S or N, and R₄₈ represents a lower alkyl or an aryl, Q₂ represents O, S or N.

A "selenoalkyl" refers to an alkyl group having a substituted seleno group attached thereto. Exemplary "selenoethers" which may be substituted on the alkyl are selected from one of -Se-alkyl, -Se-alkenyl, -Se-alkynyl, and -Se-(CH₂)ₘ-R₈, m and R₈ being defined above.

Analogous substitutions can be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The phrase "protecting group" as used herein means temporary modifications of a potentially reactive functional group which protect it from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective Groups in Organic Synthesis, 2nd ed.; Wiley: New York, 1991).

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described hereinabove. The permissible substituents can be one or more and the same or different for appropriate organic compounds. For purposes of this invention, the heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

A "polar solvent" means a solvent which has a dielectric constant (ε) of 2.9 or greater, such as DMF, THF, ethylene glycol dimethyl ether (DME), DMSO, acetone, acetonitrile, methanol, ethanol, isopropanol, n-propanol, t-butanol or 2-methoxyethyl ether. Preferred polar solvents are DMF, DME, NMP, and acetonitrile.

An "aprotic solvent" means a non-nucleophilic solvent having a boiling point range above ambient temperature, preferably from about 25°C to about 190°C, more preferably from about 80°C to about 160°C, most preferably from about 80°C to 150°C, at atmospheric pressure. Examples of such solvents are acetonitrile, toluene, DMF, diglyme, THF or DMSO.

A "polar, aprotic solvent" means a polar solvent as defined above which has no available hydrogens to exchange with the compounds of this invention during reaction, for example DMF, acetonitrile, diglyme, DMSO, or THF.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

### Exemplification

The invention may be understood with reference to the following examples, which are presented for illustrative purposes only and which are non-limiting. The substrates utilized in these examples were either commercially available, or were prepared from commercially available reagents.

### Example 1

### Pd-Catalyzed Carbon-Nitrogen Bond Formation in tert-Butanol Using (2',4',6'-tri-isopropylbiphenyl)dicyclohexylphosphine as Ligand

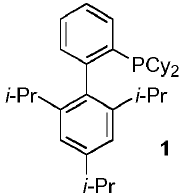

### (2',4',6'-tri-isopropylbiphenyl)dicyclohexylphosphine ("Ligand 1")

### 4-(4-Butyl-phenylamino)-benzamide

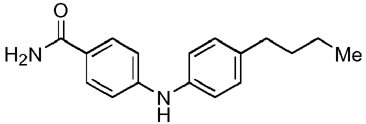

An oven-dried resealable Schlenk flask was evacuated and backfilled with argon. The flask was charged with Pd₂(dba)₃ (4.6 mg, 0.005 mmol, 1 mol % Pd), Ligand 1 (9.4 mg, 0.02 mmol, 2 mol %), ground K₂CO₃ (193 mg, 1.4 mmol), and 4-amino-benzamide (163 mg, 1.2 mmol). The flask was evacuated and backfilled with argon (3 x) and then capped with a rubber septum. To the flask was added *t*-BuOH (1.5 mL), 4-n-butyl-chlorobenzene (168 mg, 1.0 mmol) and *t*-BuOH (0.5 mL). The septum was replaced with a Teflon screwcap, the flask was sealed, and the mixture was heated to 110 °C with stirring until the starting aryl chloride was consumed by GC analysis (19 h). The reaction was cooled to room temperature, diluted with ethyl acetate, filtered through Celite, and concentrated under reduced pressure. The crude material was purified by column chromatography on silica gel (eluting with EtOAc/hexanes, 8:2) to give the desired product as a white solid (235 mg, 88 %).

The same procedure using KOH (1.4 mmol, 78 mg) in place of potassium carbonate gave an 85 % yield of the product.

### 3-(Methyl-phenyl-amino)-benzoic acid

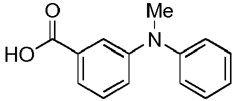

An oven-dried resealable Schlenk flask was evacuated and backfilled with argon. The flask was charged with Pd₂dba₃ (9.2 mg, 0.01 mmol, 2 mol % Pd), Ligand 1 (19 mg, 0.04 mmol, 4 mol %), pulverized KOH (168 mg, 3.0 mmol), and 3-chlorobenzoic acid (156 mg, 1.0 mmol). The flask was evacuated and backfilled with argon (3 x) and then capped with a rubber septum. To the flask was added *t*-BuOH (2.0 mL), *N*-methyl-aniline (0.163 mL, 1.5 mmol) and *t*-BuOH (0.5 mL). The septum was replaced with a Teflon screwcap, the flask was sealed, and the mixture was heated to 100 °C with stirring until the starting aryl chloride was consumed by GC analysis (3h, GC monitoring was conducted by diluting a small aliquot from the reaction with MeOH (1-2 mL), adding 2-3 drops H₂SO₄ and heating until 0.5 mL liquid was left, filtering through a small pipette silica plug and observing the disappearance of the corresponding methyl ester). The reaction was cooled to room temperature, diluted with 5 % aq. NaOH and extracted with Et₂O. The aqueous layer was cooled to 0°C and acidified with HCl (12.0 M) until pH ∼ 4. The aqueous layer was extracted with Et₂O, dried over anh. MgSO₄, filtered, and concentrated under reduced pressure to give crude product (233 mg). The product was purified by re-crystallizing in hot hexanes/chloroform (5:1) to give a light yellow solid (191 mg, 84 %, ≥95 % pure).

### 4-(4-Butyl-phenylamino)-benzoic acid

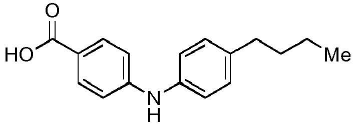

An oven-dried resealable Schlenk flask was evacuated and backfilled with argon. The flask was charged with Pd₂dba₃ (9.2 mg, 0.01 mmol, 2 mol % Pd), Ligand 1 (19 mg, 0.04 mmol, 4 mol %), pulverized KOH (168 mg, 3.0 mmol), and 4-amino-benzoic acid (165 mg, 1.2 mmol). The flask was evacuated and backfilled with argon (3 x) and then capped with a rubber septum. To the flask was added *t*-BuOH (2.0 mL), 4-n-butylchlorobenzene (168 mg, 1.0 mmol) and *t-*BuOH (0.5 mL). The septum was replaced with a Teflon screwcap, the flask was sealed, and the mixture was heated to 100 °C with stirring until the starting aryl chloride was consumed by GC analysis (3h). The reaction was cooled to room temperature, diluted with 5 % aq. NaOH and extracted with Et₂O. The aqueous layer was cooled to 0°C and acidified with HCl (12.0 M) until pH ∼ 4. The aqueous layer was extracted with Et₂O, dried over anh. MgSO₄, filtered, and concentrated under reduced pressure to give crude product (248 mg). The product was purified by re-crystallizing in hot hexanes/chloroform to give faint brown platelets (209 mg, 78 %).

### 3-(4-Methoxy-phenylamino)-benzamide

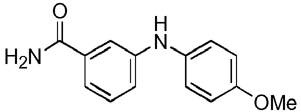

An oven-dried resealable Schlenk flask was evacuated and backfilled with argon. The flask was charged with Pd₂dba₃ (9.2 mg, 0.01 mmol, 2 mol % Pd), Ligand 1 (19 mg, 0.04 mmol, 4 mol %), K₂CO₃ (304 mg, 2.2 mmol), 3-chloro-benzamide (156 mg, 1.0 mmol), and 4-methoxy-aniline (148 mg, 1.2 mmol). The flask was evacuated and backfilled with argon (3 x) and then capped with a rubber septum. To the flask was added *t*-BuOH (2.5 mL). The septum was replaced with a Teflon screwcap, the flask was sealed, and the mixture was heated to 100 °C with stirring until the starting aryl chloride was consumed by TLC analysis (20h). The reaction was cooled to room temperature, diluted with ethyl acetate, filtered through Celite, and concentrated under reduced pressure. The crude material was purified by column chromatography on silica gel (eluting with EtOAc/hexanes, 8.5:1.5) to give the desired product which was re-crystallized from ethyl acetate/hexanes (14/1) to give white flaky crystals (191 mg, 79 %).

### Example 2

### Pd-Catalyzed Carbon-Nitrogen Bond Formation in tert-Butanol Using (2',4',6'-triisopropylbiphenyl)dicyclohexylphosphine as Ligand

### General Procedure

An oven-dried resealable Schlenk tube containing a stir bar was charged with Pd(OAc)₂ (2.3 mg, 0.01 mmol), 2-di(cyclohexyl)phosphino-2',4',6'-triisopropylbiphenyl (11.9 mg, 0.025 mmol) and phenylboronic acid (3.1 mg, 0.025 mmol). The tube was capped with a rubber septum, evacuated and backfilled with argon. *t*Butanol (0.5 mL) was added through the septum via a syringe. The reaction mixture was stirred at room temperature for 20 min. Aryl halide or sulfonate (0.5 mmol), amine (0.75 mmol) and finely ground K₂CO₃ (173 mg, 1.2 mmol) were added into the schlenk tube under a flow of argon, followed by the addition of *t*butanol (0.5 mL) via a syringe. The septum was replaced with a Teflon screw cap. The schlenk tube was sealed and put into a pre-heated oil bath at 110 °C. The reaction mixture was stirred for the time specified and the resulting mixture was allowed to cool down to room temperature, filtered through celite with ethyl acetate. The filtrate was concentrated under reduced pressure and the residue was purified by chromatography on a silica gel column.

### 4-(3,5-Dimethylphenylamino)benzamide

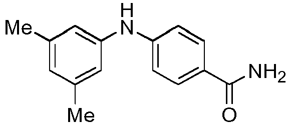

Following the general procedure, 3,5-dimethylphenyl benzenesulfonate (131 mg, 0.5 mmol) was coupled with 4-aminobenzamide (102 mg, 0.75 mmol) with the reaction time of 6 h. Chromatography on silica gel column with ethyl acetate gave 115 mg (96%) of the title compound as a white solid.

### 2-Phenylaminobenzamide

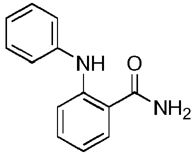

Following the general procedure, bromobenzene (55 µL, 0.5 mmol) was coupled with 2-aminobenzamide (102 mg, 0.75 mmol) with the reaction time of 15 h. Chromatography on silica gel column with 1:1 hexane : ethyl acetate gave 74 mg (70%) of the title compound as a white solid.

### 2-(4-Cyanophenylamino)benzamide

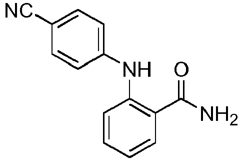

Following the general procedure, 4-bromobenzonitrile (91 mg, 0.5 mmol) was coupled with 2-aminobenzamide (102 mg, 0.75 mmol) with the reaction time of 17 h. Chromatography on silica gel column with 1:1 hexane : ethyl acetate gave 103 mg (87%) of the title compound as a pale yellow solid.

### N-(4-p-Tolylaminophenyl)acetamide

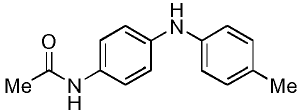

Following the general procedure, 4-chloroacetanilide (85 mg, 0.5 mmol) was coupled with *p*-toluidine (80 mg, 0.75 mmol) with the reaction temperature of 90 °C and the reaction time of 14 h. Chromatography on silica gel column with 2:1 hexane : ethyl acetate gave 118 mg (98%) of the title compound as a pink solid.

### 5-(3,5-Dimethylphenylamino)indole and 1-(3,5-dimethylphenyl)-5-aminoindole

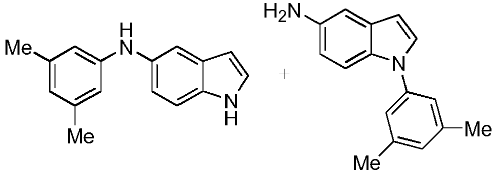

Following the general procedure, 3,5-dimethylphenyl benzenesulfonate (131 mg, 0.5 mmol) was coupled with 5-aminoindole (100 mg, 0.75 mmol) with the reaction time of 4 h. Chromatography on silica gel column with 6:1 hexane : ethyl acetate gave 88 mg (74%) of 5-(3,5-dimethylphenylamino)indole as a pale brown solid; and 16 mg (14%) of 1-(3,5-dimethylphenyl)-5-aminoindole as a white solid.

### Ethyl 4-((3-aminophenyl)amino)benzoate

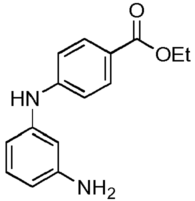

Following the general procedure, 3-bromoaniline (56 µL, 0.5 mmol) was coupled with ethyl 4-aminobenzoate (125 mg, 0.75 mmol) with the reaction temperature of 80 °C and the reaction time of 17 h. Chromatography on silica gel column with 4:1 hexane : ethyl acetate gave 55 mg (43%) of the title compound as a pale brown solid.

### N-(4-tButylphenyl)-2-pyrrolidinone

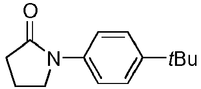

Following the general procedure, 4-*t*butylphenyl benzenesulfonate (145 mg, 0.5 mmol) was coupled with 2-pyrrolidinone (57 µL, 0.75 mmol) with the reaction time of 21 h. Chromatography on silica gel column with 1:1 hexane : ethyl acetate gave 103 mg (95%) of the title compound as a white solid.

### N-(4-tButylphenyl)acetamide

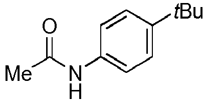

Following the general procedure, 4-*t*butylphenyl benzenesulfonate (145 mg, 0.5 mmol) was coupled with acetamide (45 mg, 0.75 mmol) with the reaction time of 21 h. Chromatography on silica gel column with 1:1 hexane : ethyl acetate gave 84 mg (88%) of the title compound as a white solid.

### N-(3,5-dimethylphenyl)-N-methylformamide

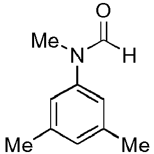

Following the general procedure, 3,5-dimethylphenyl benzenesulfonate (131 mg, 0.5 mmol) was coupled with N-methylformamide (45 mg, 0.75 mmol) with the reaction time of 20 h. Chromatography on silica gel column with 4:1 hexane : ethyl acetate gave 76 mg (93%) of the title compound as a colorless oil.

### tButyl N-(3,4-methylenedioxyphenyl)carbamate

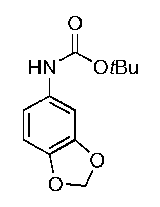

Following the general procedure, 3,4-methylenedioxyphenyl tosylate (146 mg, 0.5 mmol) was coupled with *t*butyl carbamate (90 mg, 0.75 mmol) with the reaction time of 24 h and the exception that 2-di-cyclohexylphosphino-2', 4', 6'-triisopropylbiphenyl (19.0 mg, 0.040 mmol) was used. Chromatography on silica gel column with 10:1 hexane : ethyl acetate gave 101 mg (85%) of the title compound as a white solid.

### N-(1-Naphthyl)benzamide

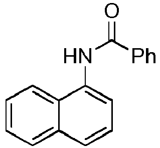

Following the general procedure, 1-naphthyl tosylate (149 mg, 0.5 mmol) was coupled with benzamide (90 mg, 0.75 mmol) with the reaction time of 20 h. Chromatography on silica gel column with 4:1 hexane : ethyl acetate gave 117 mg (95%) of the title compound as a white solid.

### Example 3

### 3-Morpholine-acetanilide

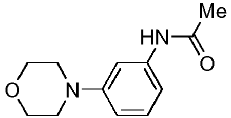

An oven-dried resealable schlenk tube containing a stir bar was charged with Pd(OAc)₂ (2.3 mg, 0.01 mmol), 2-di-cyclohexylphosphino-2', 4', 6'-triisopropylbiphenyl (11.9 mg, 0.025 mmol), 3-(N-acetyl)phenyl tosylate (153 mg, 0.5 mmol) and finely ground K₂CO₃ (173 mg, 1.2 mmol). The tube was capped with a rubber septum, evacuated and backfilled with argon. Morpholine (66 µL, 0.75 mmol) and *t*butanol (1 mL) were added through the septum via syringe. The septum was replaced with a Teflon screw cap. The schlenk tube was sealed and put into a pre-heated oil bath at 110 °C. The reaction mixture was stirred for 20 h, allowed to cool down to room temperature, filtered through celite with ethyl acetate. Chromatography on silica gel column with ethyl acetate gave 110 mg (100%) of the title compound as a white solid.

### Example 4

### N-(4-Hexylaminophenyl)acetamide

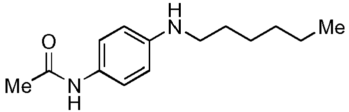

An oven-dried resealable schlenk tube containing a stir bar was charged with Pd₂(dba)₃ (4.6 mg, 0.005 mmol), 2-di-cyclohexylphosphino-2', 4', 6'-triisopropylbiphenyl (11.9 mg, 0.025 mmol), 4-bromoacetanilide (107 mg, 0.5 mmol) and sodium *t*butoxide (120 mg, 1.2 mmol). The tube was capped with a rubber septum, evacuated and backfilled with argon. *n*Hexylamine (100 µL, 0.75 mmol) and *t*butanol (1 mL) were added through the septum via syringe. The septum was replaced with a Teflon screw cap. The schlenk tube was sealed and put into a pre-heated oil bath at 110 °C. The reaction mixture was stirred for 3 h, allowed to cool down to room temperature and quenched with water. Extraction with ethyl acetate three times followed by chromatography on silica gel column with 1:1 hexane : ethyl acetate gave 95 mg (81 %) of the title compound as a white solid.

### Example 5

### α-(4-tButylphenyl)cycloheptanone

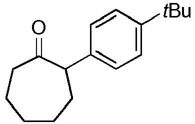

An oven-dried resealable schlenk tube containing a stir bar was charged with Pd(OAc)₂ (2.3 mg, 0.01 mmol), 2-di-cyclohexylphosphino-2', 4', 6'-triisopropylbiphenyl (11.9 mg, 0.025 mmol), 4-*t*butylphenyl benzenesulfonate (145 mg, 0.5 mmol) and Cs₂CO₃ (408 mg, 1.2 mmol). The tube was capped with a rubber septum, evacuated and backfilled with argon. Cycloheptanone (90 µL, 0.75 mmol), toluene (1 mL) and *t*-butanol (0.2 mL) were added through the septum via syringe. The septum was replaced with a Teflon screw cap. The schlenk tube was sealed and put into a pre-heated oil bath at 110 °C. The reaction mixture was stirred for 21 h, allowed to cool down to room temperature, filtered through celite with ethyl acetate. Chromatography on silica gel column with 30:1 hexane : ethyl acetate gave 104 mg (85%) of the title compound as a white solid.

### Example 6

### Diethyl (α-(4-tbutylphenyl)malonate

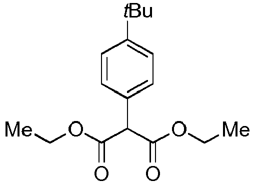

An oven-dried resealable schlenk tube containing a stir bar was charged with Pd(OAc)₂ (2.3 mg, 0.01 mmol), 2-di-cyclohexylphosphino-2', 4', 6'-triisopropylbiphenyl (11.9 mg, 0.025 mmol), 4-*t*butylphenyl benzenesulfonate (145 mg, 0.5 mmol) and Cs₂CO₃ (408 mg, 1.2 mmol). The tube was capped with a rubber septum, evacuated and backfilled with argon. Diethyl malonate (115 µL, 0.75 mmol) and toluene (1 mL) were added through the septum via syringe. The septum was replaced with a Teflon screw cap. The schlenk tube was sealed and put into a pre-heated oil bath at 110 °C. The reaction mixture was stirred for 18 h, allowed to cool down to room temperature, filtered through celite with ethyl acetate. Chromatography on silica gel column with 10:1 hexane : ethyl acetate gave 131 mg (90%) of the title compound as a colorless oil.

### Example 7

### Ethyl α-phenyl-α-(4-tbutylphenyl)acetate

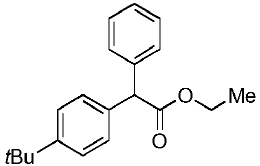

An oven-dried resealable schlenk tube containing a stir bar was charged with Pd(OAc)₂ (2.3 mg, 0.01 mmol), 2-di-cyclohexylphosphino-2', 4', 6'-triisopropylbiphenyl (11.9 mg, 0.025 mmol), 4-*t*butylphenyl benzenesulfonate (145 mg, 0.5 mmol) and Cs₂CO₃ (408 mg, 1.2 mmol). The tube was capped with a rubber septum, evacuated and backfilled with argon. Ethyl α-phenylacetate (120 µL, 0.75 mmol), toluene (1 mL) and *t*butanol (0.2 mL) were added through the septum via syringe. The septum was replaced with a Teflon screw cap. The schlenk tube was sealed and put into a pre-heated oil bath at 110 °C. The reaction mixture was stirred for 20 h, allowed to cool down to room temperature, filtered through celite with ethyl acetate. Chromatography on silica gel column with 4:1 hexane : dichloromethane gave 130 mg (88%) of the title compound as a colorless oil.

### Example 8

### Suzuki Cross-Coupling Reactions of Aryl Tosylates with Arylboronic Acids

### General Procedure

Pd(OAc)₂ (1-2 mol%), phosphine ligand (2-5 mol%), aryl tosylate (1 equiv), arylboronic acid (2 equiv) and a base (3 equiv) were added to a Schlenk tube equipped with a magnetic stir bar. The tube was evacuated and backfilled with nitrogen or argon three times. Under a nitrogen or argon atmosphere, THF (1 mL) was added via a syringe. The tube was sealed with a Teflon screwcap and heated to the specified temperature in an oil bath for the time indicated. The reaction tube was allowed to cool to room temperature when the reaction was completed according to GC, TLC and/or crude ¹H NMR. Then, the reaction was quenched with 1 mL of 1M aqueous HCl and diluted with 5 mL of EtOAc. The organic layer was separated and the aqueous layer was washed with EtOAc (2x5 mL). For pyridine and quinoline compounds, the reaction was quenched with 1 mL of water and the aqueous layer was washed repeatedly with EtOAc until all product was extracted. The combined organic layer was dried over MgSO₄. After filtering the MgSO₄, the filtrate was concentrated under reduced pressure. Column chromatography of the residue on silica gel with Hexanes:EtOAc eluant afforded the product.

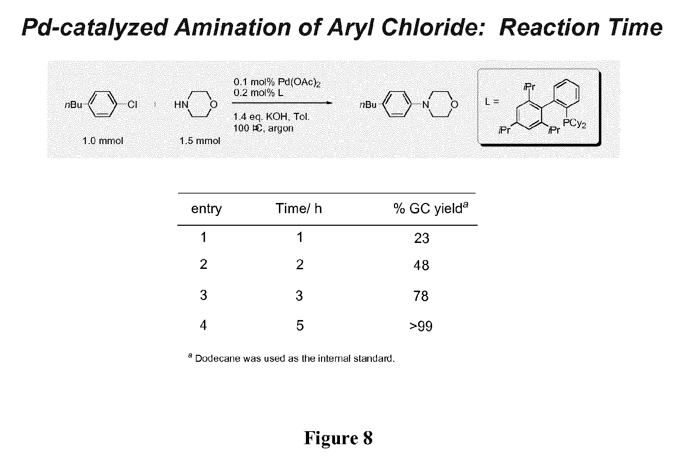

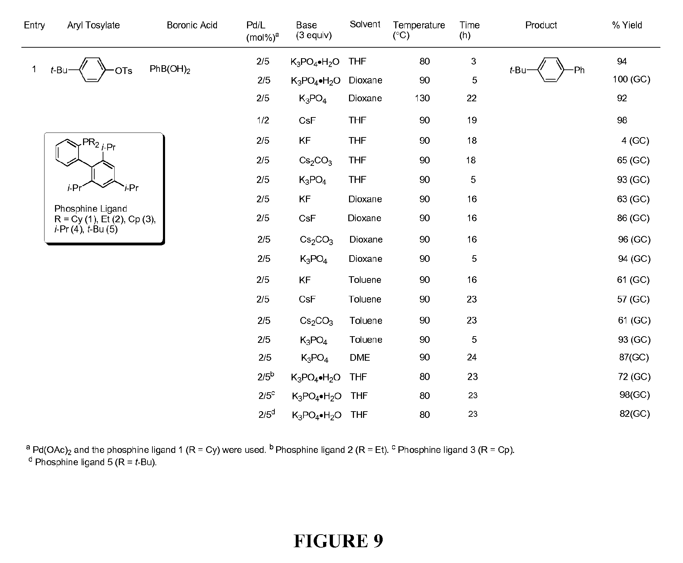

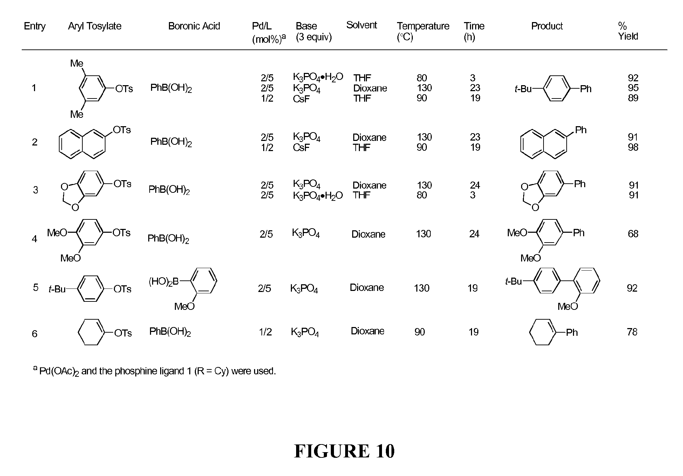

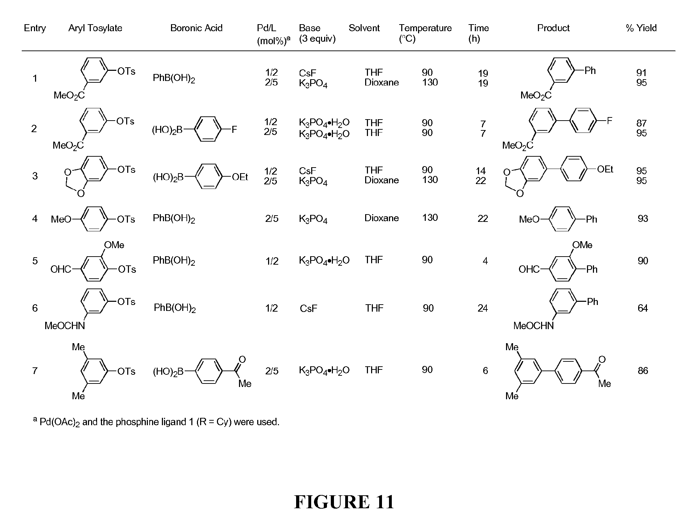

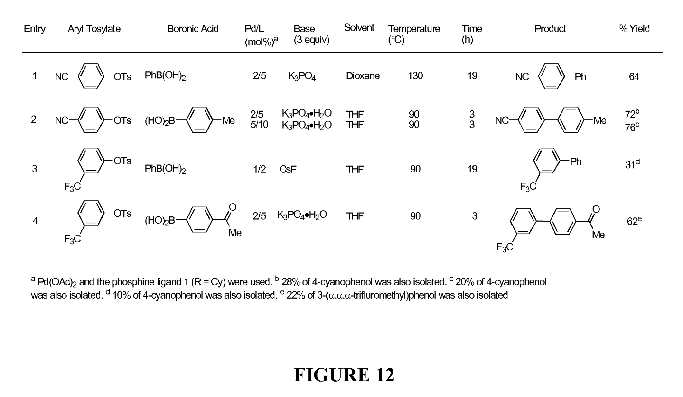

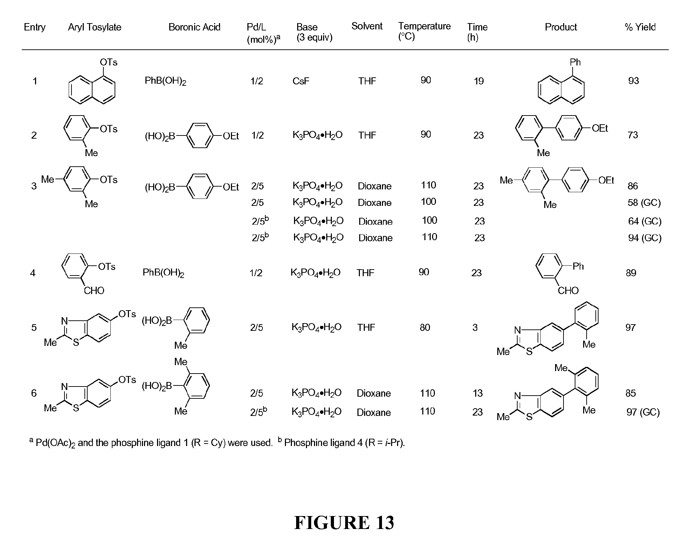

Numerous specific embodiments of the general method are presented in Figures 8-13.

### Example 9

### 5-(4-nbutylphenylamino)indole and 1 -(4-nbutylphenyl)-5-aminoindole

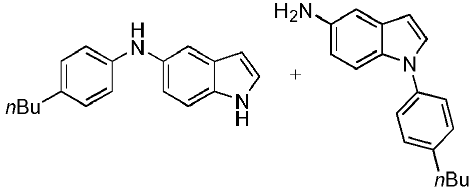

An oven-dried resealable schlenk tube containing a stir bar was charged with Pd₂(dba)₃ (4.6 mg, 0.005 mmol), 2-dicyclohexylphosphino-2', 4', 6'-triisopropylbiphenyl (11..9 mg, 0.025 mmol), 5-aminoindole (100 mg, 0.75 mmol) and K₂CO₃ (173 mg, 1.25 mmol). The tube was capped with a rubber septum, evacuated and backfilled with argon. 4-nbutylchlorobenzene (85 mg, 0.5 mmol) and *t*butanol (1 mL) were added through the septum via syringe. The septum was replaced with a Teflon screw cap. The schlenk tube was sealed and put into a pre-heated oil bath at 110 °C. The reaction mixture was stirred for 3 h, allowed to cool down to room temperature and filtered through celite with ethyl acetate. The filtrate was concentrated under reduced pressure. Chromatography on silica gel column with 4:1 hexane : ethyl acetate gave 106 mg (80%) of 5-(4-*n*butylphenylamino)indole as a pale brown solid. 1-(4-nButylphenyl)-5-aminoindole was obtained in 10% GC yield.

### Example 10 (comparative example)

### Ethyl 4-((3-aminophenyl)amino)benzoate

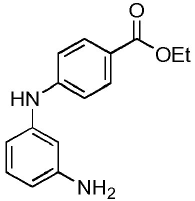

An oven-dried resealable schlenk tube containing a stir bar was charged with Pd₂(dba)₃ (4.6 mg, 0.005 mmol), 2-di*t*butylphosphino-2', 4', 6'-triisopropylbiphenyl (17.0 mg, 0.04 mmol), ethyl 4-aminobenzoate (125 mg, 0.75 mmol) and K₂CO₃ (173 mg, 1.25 mmol). The tube was capped with a rubber septum, evacuated and backfilled with argon. 3-Bromoaniline (56 µL, 0.5 mmol) and *t*butanol (1 mL) were added through the septum via syringe. The septum was replaced with a Teflon screw cap. The schlenk tube was sealed and put into a pre-heated oil bath at 80 °C. The reaction mixture was stirred for 11 h, allowed to cool down to room temperature and filtered through celite with ethyl acetate. The filtrate was concentrated under reduced pressure. Chromatography on silica gel column with 4:1 hexane : ethyl acetate gave 103 mg (80%) of the title compound as a pale yellow oil.

### Example 11

### 2-(2-Methylphenylamino)benzamide and 2-amino-N-(2-methylphenyl)benzamide

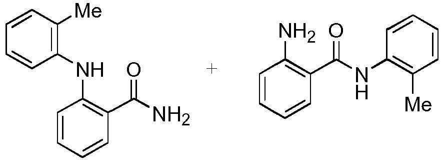

An oven-dried resealable schlenk tube containing a stir bar was charged with Pd₂(dba)₃ (4.6 mg, 0.005 mmol), 2-dicyclohexylphosphino-2', 4', 6'-triisopropylbiphenyl (19.0 mg, 0.04 mmol), 2-aminobenzamide (170 mg, 1.25 mmol) and K₂CO₃ (173 mg, 1.25 mmol). The tube was capped with a rubber septum, evacuated and backfilled with argon. 2-Chlorotoluene (60 µL, 0.5 mmol) and *t*butanol (1 mL) were added through the septum via syringe. The septum was replaced with a Teflon screw cap. The schlenk tube was sealed and put into a pre-heated oil bath at 110 °C. The reaction mixture was stirred for 17 h, allowed to cool down to room temperature and filtered through celite with ethyl acetate. The filtrate was concentrated under reduced pressure. Chromatography on silica gel column with 4:1 hexane : ethyl acetate gave 98 mg (87%) of 2-(2-methylphenylamino)benzamide as a white solid and 11 mg (10%) of 2-amino-N-(2-methylphenyl)benzamide as a white solid.

### Example 12

### N-(2-p-Tolylaminophenyl)acetamide

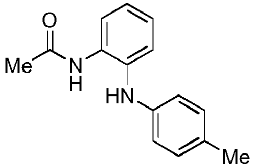

An oven-dried resealable schlenk tube containing a stir bar was charged with Pd₂(dba)₃ (4.6 mg, 0.005 mmol), 2-dicyclohexylphosphino-2', 4', 6'-triisopropylbiphenyl (19.0 mg, 0.04 mmol), 2-bromoacetanilide (107 mg, 0.5 mmol), *p*-toluidine (80 mg, 0.75 mmol) and K₃PO₄ (265 mg, 1.25 mmol). The tube was capped with a rubber septum, evacuated and backfilled with argon. *t*Butanol (1 mL) was added through the septum via syringe. The septum was replaced with a Teflon screw cap. The schlenk tube was sealed and put into a pre-heated oil bath at 110 °C. The reaction mixture was stirred for 18 h, allowed to cool down to room temperature and filtered through celite with ethyl acetate. The filtrate was concentrated under reduced pressure. Chromatography on silica gel column with 1:1 hexane : ethyl acetate gave 107 mg (89%) of the title compound as a brown solid.

### Example 13

### N-(3-Dibutylaminophenyl)acetamide

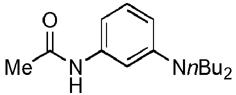

An oven-dried resealable schlenk tube containing a stir bar was charged with Pd₂(dba)₃ (4.6 mg, 0.005 mmol), 2-dicyclohexylphosphino-2', 4', 6'-triisopropylbiphenyl (11.9mg, 0.025 mmol), 3-chloroacetanilide (85 mg, 0.5 mmol) and sodium *t*butoxide (120 mg, 1.25 mmol). The tube was capped with a rubber septum, evacuated and backfilled with argon. Di*n*butylamine (126 µL, 0.75 mmol) and *t*butanol (1 mL) were added through the septum via syringe. The septum was replaced with a Teflon screw cap. The schlenk tube was sealed and put into a pre-heated oil bath at 90 °C. The reaction mixture was stirred for 18 h, allowed to cool down to room temperature and quenched with water. Extraction with ethyl acetate three times followed by chromatography on silica gel column with 4:1 hexane : ethyl acetate gave 106 mg (81 %) of the title compound as a pale yellow oil.

### Example 14

### 2-Phenylaminobenzamide and 2-amino-N-phenylbenzamide using t-amyl alcohol as solvent

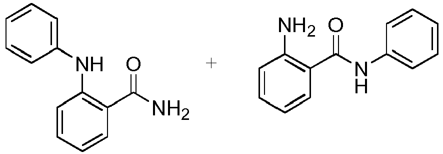

An oven-dried resealable schlenk tube containing a stir bar was charged with Pd₂(dba)₃ (4.6 mg, 0.005 mmol), 2-dicyclohexylphosphino-2', 4', 6'-triisopropylbiphenyl (19.0 mg, 0.04 mmol), 2-aminobenzamide (102 mg, 0.75 mmol) and K₂CO₃ (173 mg, 1.25 mmol). The tube was capped with a rubber septum, evacuated and backfilled with argon. Bromobenzene (55 µL, 0.5 mmol) and *t*amyl alcohol (1 mL) were added through the septum via syringe. The septum was replaced with a Teflon screw cap. The schlenk tube was sealed and put into a pre-heated oil bath at 110 °C. The reaction mixture was stirred for 15 h, allowed to cool down to room temperature. GC analysis showed full conversion of bromobenzene. 2-Phenylaminobenzamide was obtained in 65% GC yield and 2-amino-N-phenylbenzamide in 10% GC yield.

### Example 15 (comparative example)

### N-(4-Hexylaminophenyl)acetamide using PtBu₃ as the ligand

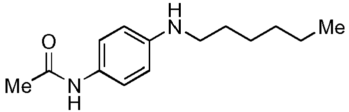

An oven-dried resealable schlenk tube containing a stir bar was charged with Pd₂(dba)₃ (4.6 mg, 0.005 mmol) and 4-bromoacetanilide (107 mg, 0.5 mmol). The tube was capped with a rubber septum, evacuated and backfilled with argon. The tube was capped with a rubber septum, evacuated and backfilled with argon. The septum was replaced with a Teflon screw cap and the tube was brought into the glovebox. The tube was charged with tri*t*butylphosphine (5.1 mg, 0.025 mmol) and sodium *t*butoxide (120 mg, 1.25 mmol) and sealed with Teflon screw cap. The tube was brought out of the glovebox and the Teflon screw cap was replaced with a rubber septum under a flow of argon. *n*Hexylamine (100 µL, 0.75 mmol) and *t*butanol (1 mL) were added through the septum via syringe. The septum was replaced with a Teflon screw cap. The schlenk tube was sealed and put into a pre-heated oil bath at 110 °C. The reaction mixture was stirred for 3 h, allowed to cool down to room temperature and quenched with water. The aqueous layer was extracted with ethyl acetate three times. GC analysis of the organics showed the title compound was obtained in 25% GC yield.

### Example 16

### Pd-Complex-Catalyzed Aminations in Water using an Aryl Chloride and Potassium Hydroxide

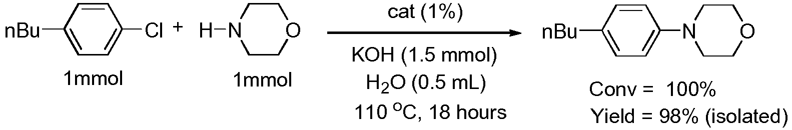

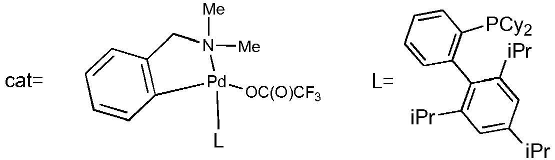

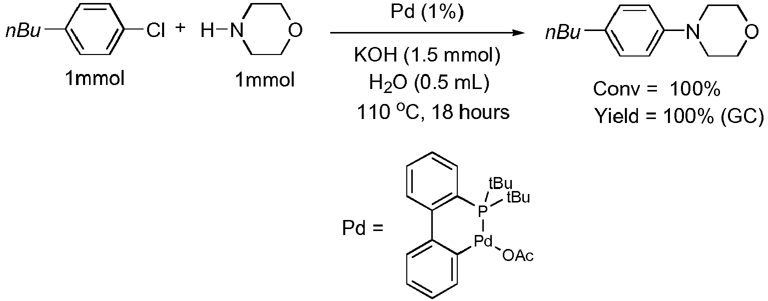

A resealable Schlenk flask was charged with palladium complex (0.01 mmol) and KOH (86 mg, 1.5 mmol). The flask was evacuated, backfilled with argon and then, to it, were added 4-n-butylchlorobenzene (1 mmol), morpholine (1.2 mmol) and deionized, degassed water (0.5 mL). The flask was sealed with a teflon screwcap and the mixture was stirred at 110 °C for 18 h. After all starting material had been consumed, as judged by GC, the mixture was allowed to cool to room temperature and then was diluted with ether (40 mL). The resulting suspension was transferred to a separatory funnel and washed with water (10 mL). The organic layer was separated, dried with MgSO₄ and concentrated under vacuum. The crude material was purified by flash chromatography on silica gel.

### Example 17

### Pd-Complex-Catalyzed Amination in Water using an Aryl Chloride and Potassium Hydroxide

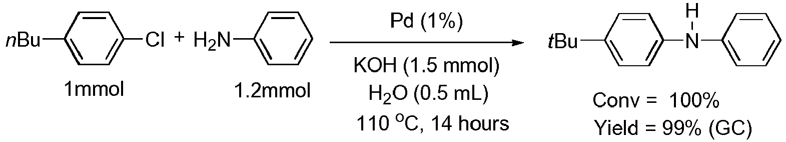

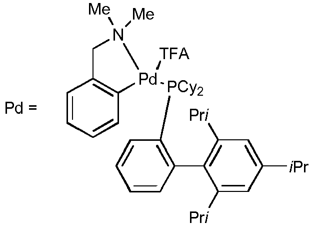

An oven-dried resealable Schlenk flask was charged with palladium complex (8.3 mg, 0.01 mmol), and KOH (84 mg, 1.5 mmol). The flask was evacuated, backfilled with argon and then, to it, were added 4-n-butylchlorobenzene (170 µL, 1 mmol), aniline (109 µL, 1.2 mmol), dodecane as internal standard (15 mg) and water (0.5 mL). The flask was sealed with a teflon screwcap and the mixture was stirred at 110 °C for 14 hours. After this time the reaction was cooled to room temperature and the conversion and the yield were determined by gas chromatography using dodecane as an internal standard.

### Example 18

### Pd-Complex-Catalyzed Amination in Water using an Aryl Chloride and Potassium Hydroxide

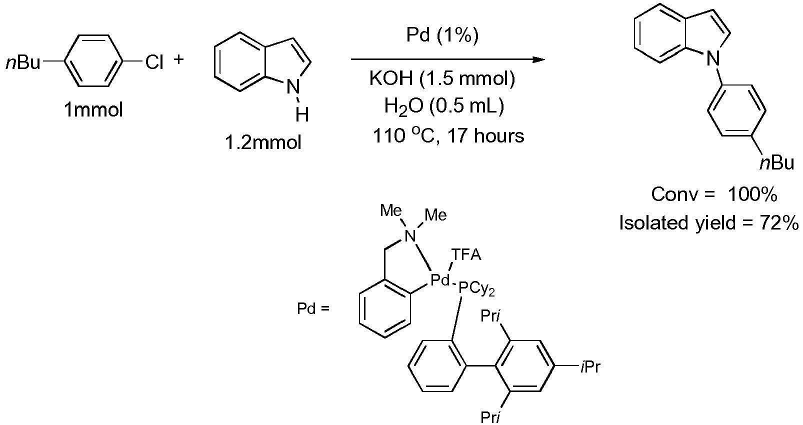

An oven-dried resealable Schlenk flask was charged with palladium complex (8.3 mg, 0.01 mmol), KOH (86 mg, 1.5 mmol) and indole (140 mg, 1.2 mmol). The flask was evacuated, backfilled with argon and then, to it, were added 4-n-butylchlorobenzene (170 µL 1 mmol), and *t*-water (0.5 mL). The flask was sealed with a teflon screwcap and the mixture was stirred at 110 °C for 17 hours. After all starting material had been consumed, as judged by GC, the mixture was allowed to cool to room temperature and then was diluted with ether (40 mL). The resulting suspension was transferred to a separatory funnel and washed with water (10 mL). The organic layer was separated, dried with MgSO₄ and concentrated under vacuum. The crude material was purified by flash chromatography on silica gel.

### Example 19

### Pd-Complex-Catalyzed Amination in Water using an Aryl Chloride and Potassium Hydroxide

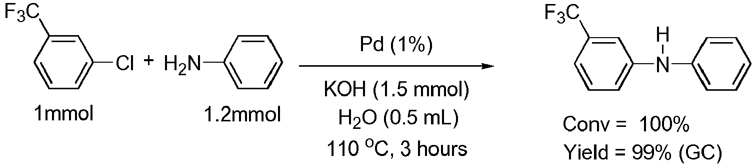

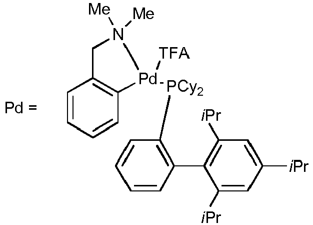

A resealable Schlenk flask was charged with palladium complex (8.3 mg, 0.01 mmol), and KOH (84 mg, 1.5 mmol). The flask was evacuated, backfilled with argon and then, to it, were added 3-trifluoromethylchlorobenzene (163 µL, 1 mmol), aniline (109 µL, 1.2 mmol), dodecane as internal standard (15 mg) and water (0.5 mL). The flask was sealed with a teflon screwcap and the mixture was stirred at 110 °C for 3 hours. After this time the reaction was cooled to room temperature and the conversion and the yield were determined by gas chromatography using dodecane as an internal standard.

### Example 20

### Pd-Complex-Catalyzed Amination in Water using an Aryl Chloride and Potassium Hydroxide

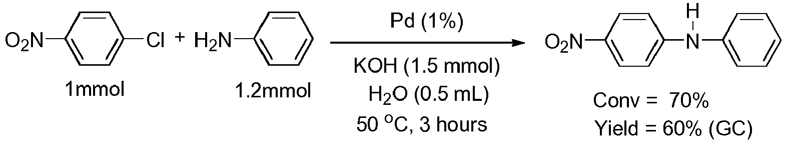

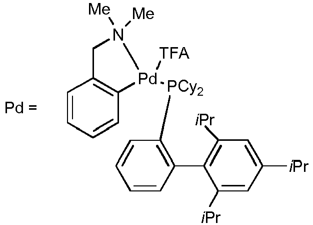

A resealable Schlenk flask was charged with palladium complex (8.3 mg, 0.01 mmol), 4-chloronitrobenzene (158 mg, 1 mmol), and KOH (84 mg, 1.5 mmol). The flask was evacuated, backfilled with argon and then, to it, were added aniline (109 µL, 1.2 mmol), dodecane as internal standard (15 mg) and water (0.5 mL). The flask was sealed with a teflon screwcap and the mixture was stirred at 50 °C for 3 hours. After this time the reaction was cooled to room temperature and the conversion and the yield were determined by gas chromatography using dodecane as an internal standard.

### Example 21

### Pd-Complex-Catalyzed Amination in Water using an Aryl Chloride and Potassium Hydroxide

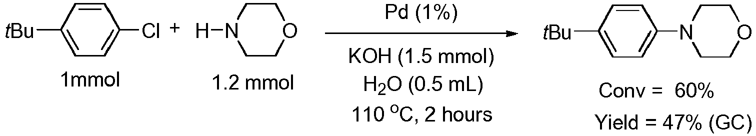

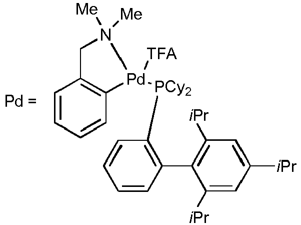

A resealable Schlenk flask was charged with palladium complex (8.3 mg, 0.01 mmol), and KOH (84 mg, 1.5 mmol). The flask was evacuated, backfilled with argon and then, to it, were added 4-n-butylchlorobenzene (170 µL, 1 mmol), morpholine (104 µL, 1.2 mmol), dodecane as internal standard (15 mg) and water (0.5 mL). The flask was sealed with a teflon screwcap and the mixture was stirred at 110 °C for 2 hours. After this time the reaction was cooled to room temperature and the conversion and the yield were determined by gas chromatography using dodecane as an internal standard.

### Example 22

### Pd-Complex-Catalyzed Amination in Water using an Aryl Chloride and Potassium Hydroxide

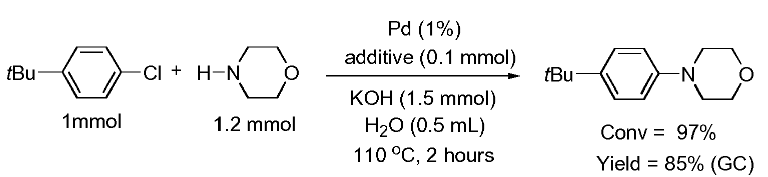

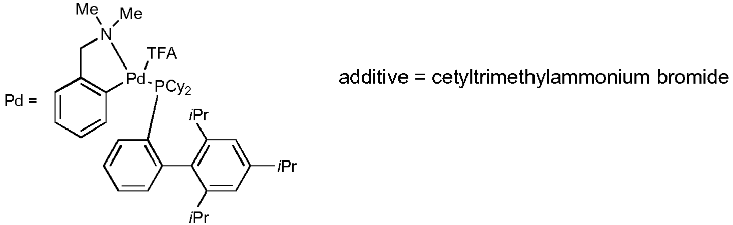

A resealable Schlenk flask was charged with palladium complex (8.3 mg, 0.01 mmol), cetyltrimethylammonium bromide (36.4 mg, 0.1 mmol) and KOH (84 mg, 1.5 mmol). The flask was evacuated, backfilled with argon and then, to it, were added 4-n-butylchlorobenzene (170 µL, 1 mmol), morpholine (104 µL, 1.2 mmol), dodecane as internal standard (15 mg) and water (0.5 mL). The flask was sealed with a teflon screwcap and the mixture was stirred at 110 °C for 2 hours. After this time the reaction was cooled to room temperature and the conversion and the yield were determined by gas chromatography using dodecane as an internal standard.

### Example 23 (comparative example)

### Pd-Complex-Catalyzed Amination in Water using an Aryl Chloride and Potassium Carbonate

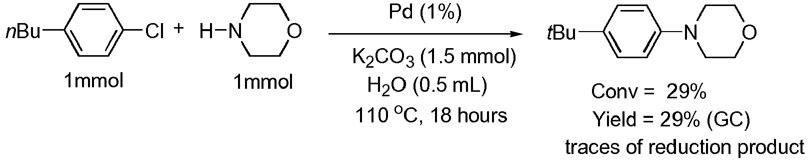

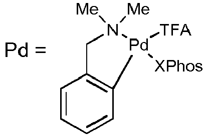

A resealable Schlenk flask was charged with palladium complex (0.01 mmol) and K₂CO₃ (207 mg, 1.5 mmol). The flask was evacuated, backfilled with argon and then, to it, were added 4-n-butylchlorobenzene (1 mmol), morpholine (1.2 mmol) and degassed. deionized water (0.5 mL). The flask was sealed with a teflon screwcap and the mixture was stirred at 110 °C for 18 hours. After all starting material had been consumed, as judged by GC, the mixture was allowed to cool to room temperature and then was diluted with ether (40 mL). The resulting suspension was transferred to a separatory funnel and washed with water (10 mL). The organic layer was separated, dried with MgSO₄ and concentrated under vacuum. The crude material was purified by flash chromatography on silica gel.

### Example 24

### Pd-Complex-Catalyzed Amination in Water using an Aryl Chloride and Sodium Hydroxide

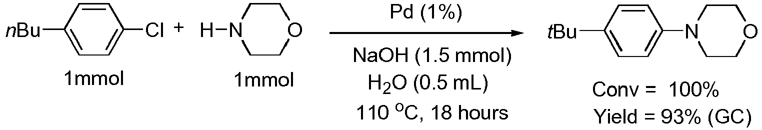

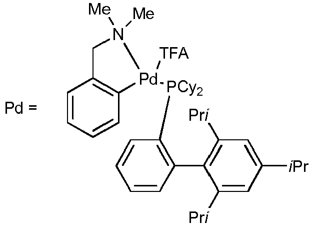

An oven-dried resealable Schlenk flask was charged with palladium complex (8.3 mg, 0.01 mmol), and NaOH (60 mg, 1.5 mmol). The flask was evacuated, backfilled with argon and then, to it, were added 4-n-butylchlorobenzene (170 µL, 1 mmol), morpholine (104 µL, 1.2 mmol), dodecane as internal standard (15 mg) and water (0.5 mL). The flask was sealed with a teflon screwcap and the mixture was stirred at 110 °C for 17 hours. After this time the reaction was cooled to room temperature and the conversion and the yield were determined by gas chromatography using dodecane as an internal standard.

### Example 25 (comparative example)

### Pd-Complex-Catalyzed Amination in Water using an Aryl Chloride and Sodium Hydroxide

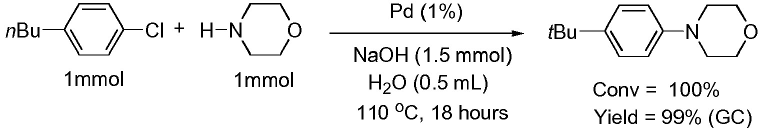

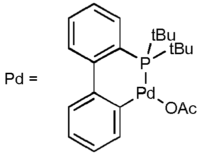

An oven-dried resealable Schlenk flask was charged with palladium complex (4.6 mg, 0.01 mmol), and NaOH (60 mg, 1.5 mmol). The flask was evacuated, backfilled with argon and then, to it, were added 4-n-butylchlorobenzene (170 µL, 1 mmol), morpholine (104 µL, 1.2 mmol), dodecane as internal standard (15 mg) and water (0.5 mL). The flask was sealed with a teflon screwcap and the mixture was stirred at 110 °C for 17 hours. After this time the reaction was cooled to room temperature and the conversion and the yield were determined by gas chromatography using dodecane as an internal standard.

### Example 26

### Pd₂(dba)₃-Catalyzed Amination in Water using an Aryl Chloride and Potassium Hydroxide

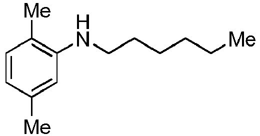

A resealable schlenk flask was evacuated and backfilled with argon. The flask was charged with Pd₂(dba)₃ (4.6 mg, 0.005 mmol, 1 mol% Pd), 2-dicyclohexylphosphino-2',4',6'-triisopropylbiphenyl (9.5 mg, 0.02 mmol, 2 mol%), and powdered KOH (78 mg, 1.4 mmol). The flask was evacuated and backfilled with argon (3 x) and capped with a rubber septum. 2-Chloro-*p*-xylene (0.134 mL, 1.0 mmol), n-hexyl-amine (0.58 mL, 1.2 mmol), and degassed water (0.5 mL) were added through the septum. The septum was replaced with a Teflon screwcap, the flask was sealed, and the mixture was heated to 110°C with stirring. At 2 h, 54 % of the aryl halide had been consumed with no further conversion upon re-heating (50 % GC yield).

### Example 27

### Pd₂(dba)₃-Catalyzed Amination in Water using an Aryl Chloride and Potassium Hydroxide

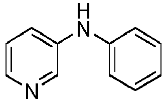

A resealable schlenk flask was evacuated and backfilled with argon. The flask was charged with Pd₂(dba)₃ (4.6 mg, 0.005 mmol, 1 mol% Pd), 2-dicyclohexylphosphino-2',4',6'-triisopropylbiphenyl (9.5 mg, 0.02 mmol, 2 mol%), and powdered KOH (78 mg, 1.4 mmol). The flask was evacuated and backfilled with argon (3 x) and capped with a rubber septum. 3-Chloro-pyridine (0.095 mL, 1.0 mmol), aniline (0.109 mL, 1.2 mmol), and degassed water (0.5 mL) were added through the septum. The septum was replaced with a Teflon screwcap, the flask was sealed, and the mixture was heated to 110°C with stirring until the starting aryl halide had been consumed by GC analysis (6 h). After complete consumption of the aryl halide, the reaction was cooled to room temperature, diluted with ethyl acetate, filtered through celite and concentrated under reduced pressure. The crude material was purified by column chromatography on silica gel (eluting with 70 % ethyl acetate in hexanes) to give the desired product as a white solid (162 mg, 95 %).

### Example 28

### Pd(II) Acetate-Catalyzed Amination in Water using an Aryl Chloride and Potassium Hydroxide

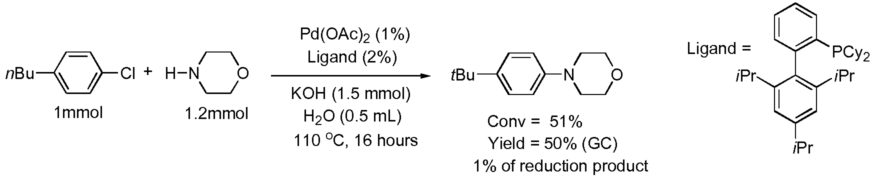

A resealable Schlenk flask was charged with palladium acetate (2.2 mg, 0.01 mmol), ligand (9.5 mg, 0.02 mmol) and KOH (86 mg, 1.5 mmol). The flask was evacuated, backfilled with argon. Then, 4-n-butylchlorobenzene (170 mL, 1 mmol), morpholine (104 mL, 1.2 mmol), dodecane (as internal standard; 15 mg) and water (0.5 mL) were added. The flask was sealed with a teflon screwcap and the mixture was stirred at 110°C for 16 hours. The reaction was allowed to cool to room temperature and the conversion and the yield were determined by gas chromatography using dodecane as an internal standard.

### Example 29

### Pd(II) Acetate-Catalyzed Amination in Water using an Aryl Chloride and Sodium Hydroxide

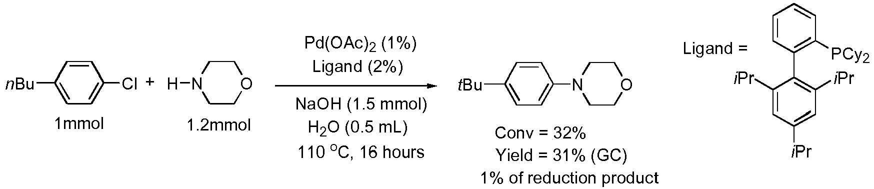

A resealable Schlenk flask was charged with palladium acetate (2.2 mg, 0.01 mmol), ligand (9.5 mg, 0.02 mmol) and NaOH (60 mg, 1.5 mmol). The flask was evacuated, backfilled with argon. Then, 4-n-butylchlorobenzene (170 mL, 1 mmol), morpholine (104 mL, 1.2 mmol), dodecane (internal standard; 15 mg) and water (0.5 mL) were added. The flask was sealed with a teflon screwcap and the mixture was stirred at 110°C for 16 hours. The reaction was allowed to cool to room temperature and the conversion and the yield were determined by gas chromatography using dodecane as an internal standard.

### Example 30

### Pd(II) Acetate-Catalyzed Aminations in Water using an Aryl Bromide and Potassium Hydroxide using Various Ligands

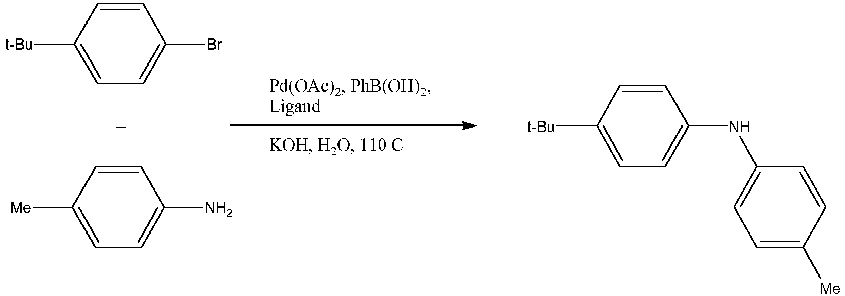

Ligands Used

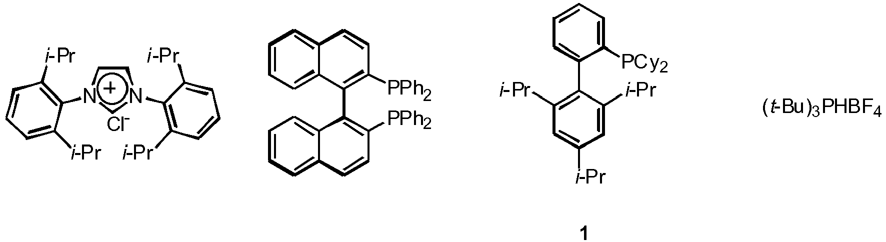

4-Toluidine (129 mg, 1.2 mmol), Pd(OAc)₂, (2.3 mg, 0.01 mmol), ligand (0.025 mmol), and KOH (78 mg 1.4 mmol) were charged to a Schlenk tube. The tube was pumped and refilled with argon twice; then, 4-*t*-butyl bromobenzene (213 mg, 0.177 mL, 1 mmol) and water (0.5 mL) were added. The resulting mixture was refluxed at 110 °C for 12 h. The products were analyzed by GC analysis (with dodecane as internal standard).

The reaction was performed using BINAP as ligand (15 mg). GC conversion: 67%, GC yield 65%.

The reaction was performed using tri-*tert*-butylphosphine as ligand, generated *in situ* using tri-*t*-butylphosphonium tetrafluoroborate (8 mg), which reacts with the KOH: GC Conversion 99%, GC yield 97%.

The reaction was performed using ligand 1 (12 mg), GC Conversion 99%, GC yield 98%.

The reaction was performed using 1,3-Bis(2,6-di-*iso*-propylphenyl)-4,5-dihydro-imidazolium tetrafluoroborate, GC Conversion 10%, GC yield 5%.

### Example 31

### Pd(II) Acetate-Catalyzed Aminations in Water using an Aryl Bromide and Tributylamine using Various Ligands

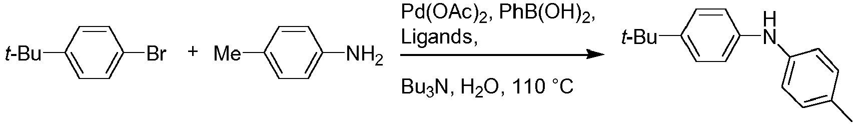

Ligands Used

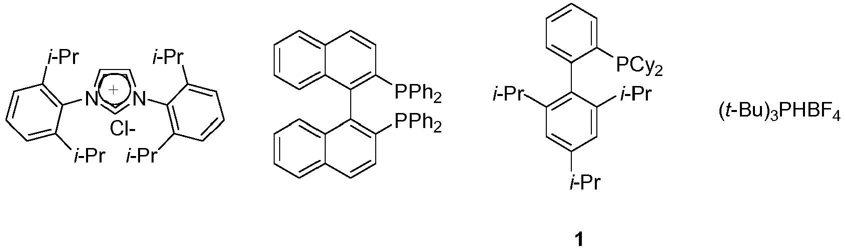

4-Toluidine (129 mg, 1.2 mmol), Pd(OAc)₂, (2.3 mg, 0.01 mmol), and a ligand (0.025 mmol), were charged to a Schlenk tube. The tube was pumped and refilled with argon twice; then, 4-*t*-butyl bromobenzene (213 mg, 0.177 mL, 1 mmol), tributylamine (0.332 mL mg 1.4 mmol), and water (0.5 mL) were added. The resulting mixture was refluxed at 110 °C for 3 h. The products were analyzed by GC analysis (with dodecane as internal standard).

The reaction was performed using BINAP as ligand (15 mg). GC conversion: 1%, GC yield less than 1%.

The reaction was performed using tri-*t*-butylphosphonium tetrafluoroborate as ligand (8 mg). GC Conversion 2%, GC yield 1%.

The reaction was performed using ligand 1 (12 mg). GC Conversion 3%, GC yield 2%.

The reaction was performed using 1,3-bis(2,6-di-*iso*-propylphenyl)-4,5-dihydro-imidazolium tetrafluoroborate. GC Conversion less than 1%, GC yield less than 1%.

### Example 32

### Pd(II) Acetate-Catalyzed Aminations in Water using an Aryl Bromide and Potassium Hydroxide using Various Ligands without using Phenylboronic Acid

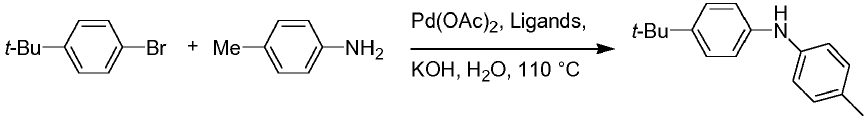

Ligands Used

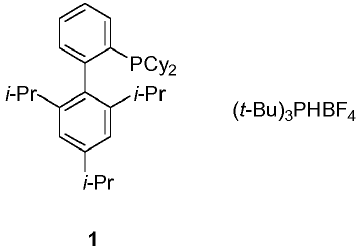

4-Toluidine (129 mg, 1.2 mmol), Pd(OAc)₂, (2.3 mg, 0.01 mmol), a ligand (0.025 mmol), and KOH (78 mg 1.4 mmol) were charged to a Schlenk tube. The tube was pumped and refilled with argon twice. Then, 4-*t*-butyl bromobenzene (213 mg, 0.177 mL, 1 mmol) and water (0.5 mL) were added. The resulting mixture was refluxed at 110 °C for 12 h. The products were analyzed by GC analysis (using dodecane as internal standard).

The reaction was performed using tri-*t*-butylphosphonium tetrafluoroborate as ligand (8 mg). GC Conversion 99%, GC yield 85%.

The reaction was performed using ligand 1 (12 mg). GC Conversion 99%, GC yield 98%.

### Example 33

### Pd(II) Acetate-Catalyzed Aminations in Water using an Aryl Bromide and Sodium Hydroxide using Various Ligands

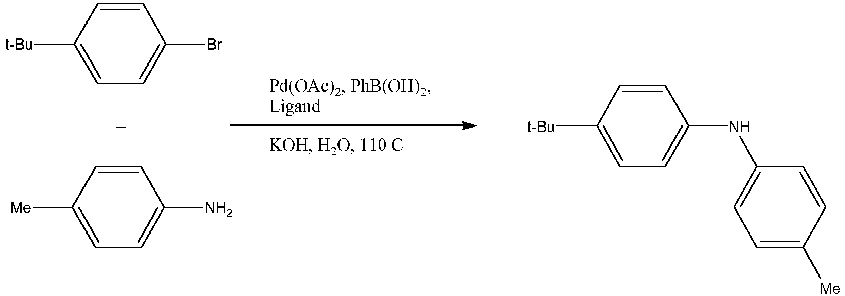

Ligands Used

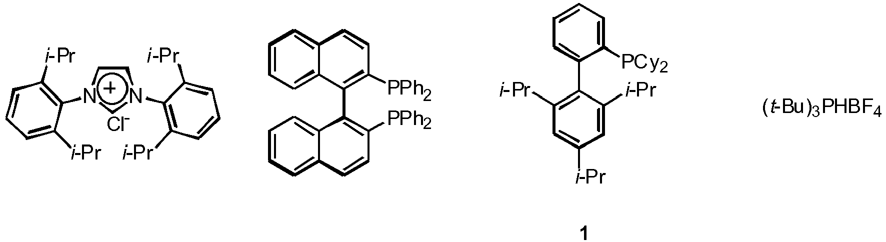

4-Toluidine (129 mg, 1.2 mmol), Pd(OAc)₂, (2.3 mg, 0.01 mmol), ligand (0.025 mmol), and NaOH (78 mg, 1.4 mmol) were charged to a Schlenk tube. The tube was pumped and refilled with argon twice; then, 4-*t*-butyl bromobenzene (213 mg, 0.177 mL, 1 mmol) and water (0.5 mL) were added. The resulting mixture was refluxed at 110 °C for 3 h. The products were analyzed by GC analysis (with dodecane as internal standard).

The reaction was performed using BINAP as ligand (15 mg). GC conversion: 4%, GC yield 2%.

The reaction was performed using tri-*tert*-butylphosphonium tetrafluoroborate as ligand (8 mg), GC Conversion 99%, GC yield 97%.

The reaction was performed using ligand 1 (12 mg), GC Conversion 99%, GC yield 98%.

The reaction was performed using 1,3-Bis(2,6-di-*iso*-propylphenyl)-4,5-dihydro-imidazolium tetrafluoroborate, GC Conversion 1%, GC yield 1%.

### Example 34 (comparative example)

### Pd-Complex-Catalyzed Amination in tert-Butanol using an Aryl Chloride and Potassium Carbonate

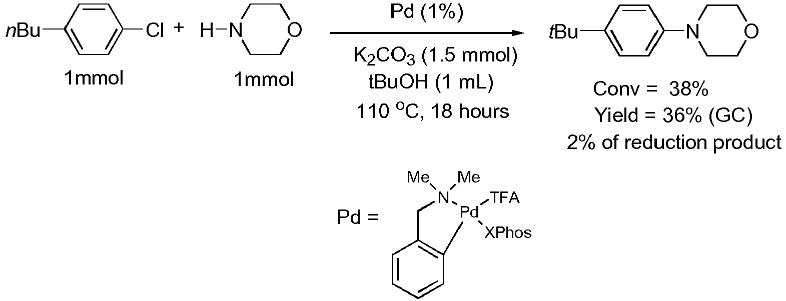

An oven-dried resealable Schlenk flask was charged with palladium complex (0.01 mmol) and K₂CO₃ (207 mg, 1.5 mmol). The flask was evacuated, backfilled with argon and then, to it, were added 4-n-butylchlorobenzene (1 mmol), morpholine (1.2 mmol) and *t*-buthanol (1 mL). The flask was sealed with a teflon screwcap and the mixture was stirred at 110°C for 18 hours. After all starting material had been consumed, as judged by GC, the mixture was allowed to cool to room temperature and then was diluted with ether (40 mL). The resulting suspension was transferred to a separatory funnel and washed with water (10 mL). The organic layer was separated, dried with MgSO₄ and concentrated under vacuum. The crude material was purified by flash chromatography on silica gel.

### Example 35 (comparative example)

### Pd-Complex-Catalyzed Amination in tert-Butanol using an Aryl Chloride and Potassium Hydroxide

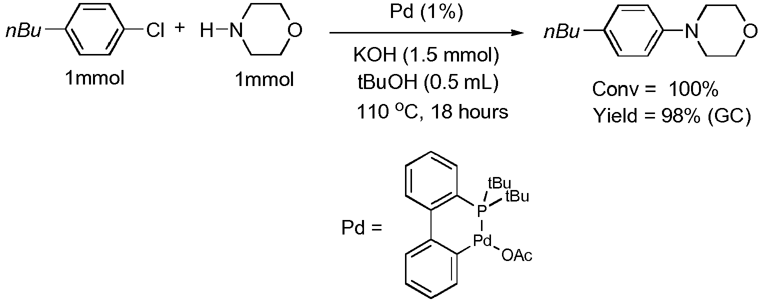

An oven-dried resealable Schlenk flask was charged with palladium complex (0.01 mmol) and KOH (86 mg, 1.5 mmol). The flask was evacuated, backfilled with argon and then, to it, were added 4-n-butylchlorobenzene (1 mmol), morpholine (1.2 mmol) and *t*-butanol (1 mL). The flask was sealed with a teflon screwcap and the mixture was stirred at 110°C for 18 h. After all starting material had been consumed, as judged by GC, the mixture was allowed to cool to room temperature and then was diluted with ether (40 mL). The resulting suspension was transferred to a separatory funnel and washed with water (10 mL). The organic layer was separated, dried with MgSO₄ and concentrated under vacuum. The crude material was purified by flash chromatography on silica gel.

## Claims

1. A ligand represented by structure II: 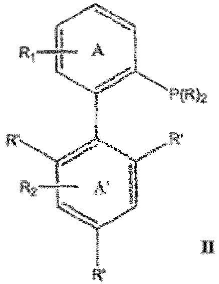 wherein
R is independently for each occurrence cycloalkyl;
wherein R' represents isopropyl;
the A and A' rings of the biphenyl core independently may be unsubstituted or substituted with R₁ and R₂, respectively, any number of times up to the limitations imposed by stability and the rules of valence;
R₁ and R₂, when present, are selected independently for each occurrence from the group consisting of alkyl, cycloalkyl, halogen, -SiR₃, and -(CH₂)ₘ-R₈₀;
R₈₀ represents independently for each occurrence cycloalkyl or aryl;
m is independently for each occurrence an integer in the range 0 to 8 inclusive; and
the ligand, when chiral, is a mixture of enantiomers or a single enantiomer.

2. The ligand of claim 1, wherein R₁ is absent; and R₂ is absent.

3. The ligand of claim 1, wherein R represents independently for each occurrence cyclohexyl or cyclopropyl.

4. The ligand of claim 1, wherein R represents independently for each occurrence cyclohexyl.

5. The ligand of claim 1, wherein R₁ is absent; R₂ is absent; and R represents independently for each occurrence cyclohexyl or cyclopropyl.

6. The ligand of claim 1, wherein R₁ is absent; R₂ is absent; and R represents cyclohexyl.

## Patentansprüche

1. Ligand, repräsentiert durch Struktur II: 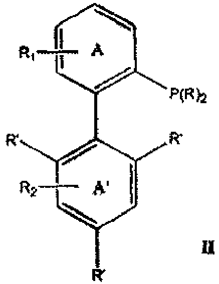 wobei
R unabhängig für jedes Auftreten Cycloalkyl ist; wobei R' Isopropyl repräsentiert;
die A- und A'-Ringe des Biphenylkerns mit R₁ bzw. R₂ unabhängig mit einer beliebigen Häufigkeit bis zu den Grenzen, die durch Stabilität und die Valenzregeln auferlegt werden, unsubstituiert oder substituiert sein können;
R₁ und R₂, wenn vorhanden, unabhängig für jedes Auftreten aus der Gruppe bestehend aus Alkyl, Cycloalkyl, Halogen, -SiR₃ und -(CH₂)ₘ-R₈₀ ausgewählt werden;
R₈₀ unabhängig für jedes Auftreten Cycloalkyl oder Aryl repräsentiert;
m unabhängig für jedes Auftreten eine ganze Zahl im Bereich von 0 bis einschließlich 8 ist; und
der Ligand, wenn chiral, ein Gemisch von Enantiomeren oder eines einzelnen Enantiomers ist.

2. Ligand nach Anspruch 1, wobei R₁ fehlt; und R₂ fehlt.

3. Ligand nach Anspruch 1, wobei R unabhängig für jedes Auftreten Cyclohexyl oder Cyclopropyl repräsentiert.

4. Ligand nach Anspruch 1, wobei R unabhängig für jedes Auftreten Cyclohexyl repräsentiert.

5. Ligand nach Anspruch 1, wobei R₁ fehlt; und R₂ fehlt; und R unabhängig für jedes Auftreten Cyclohexyl oder Cyclopropyl repräsentiert.

6. Ligand nach Anspruch 1, wobei R₁ fehlt; und R₂ fehlt; und R unabhängig Cyclohexyl repräsentiert.

## Revendications

1. Ligand représenté par la structure II : 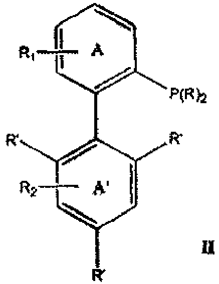 dans laquelle
R représente indépendamment pour chaque occurrence un groupe cycloalkyle ;
R' représente un groupe isopropyle ;
les cycles A et A' du noyau biphényle peuvent être indépendamment non substitués ou substitués par R₁ et R₂, respectivement, un nombre quelconque de fois jusqu'aux limites imposées par la stabilité et les règles de valence ;
R₁ et R₂, lorsqu'ils sont présents, sont choisis indépendamment pour chaque occurrence dans le groupe constitué par un groupe alkyle, cycloalkyle, un atome d'halogène, un groupe -SiR₃ et -(CH₂)ₘ-R₈₀ ;
R₈₀ représente indépendamment pour chaque occurrence un groupe cycloalkyle ou aryle ;
m représente indépendamment pour chaque occurrence un entier compris dans la plage de 0 à 8 inclus ; et
le ligand, lorsqu'il est chiral, est un mélange d'énantiomères ou un seul énantiomère.

2. Ligand selon la revendication 1, R₁ étant absent et R₂ étant absent.

3. Ligand selon la revendication 1, R représentant indépendamment pour chaque occurrence un groupe cyclohexyle ou cyclopropyle.

4. Ligand selon la revendication 1, R représentant indépendamment pour chaque occurrence un groupe cyclohexyle.

5. Ligand selon la revendication 1, R₁ étant absent, R₂ étant absent et R représentant indépendamment pour chaque occurrence un groupe cyclohexyle ou cyclopropyle.

6. Ligand selon la revendication 1, R₁ étant absent, R₂ étant absent et R représentant un groupe cyclohexyle.
